# EUROPÄISCHE PATENTSCHRIFT

(11) **EP 2 368 998 B1**
(45) Veröffentlichungstag und Bekanntmachung des Hinweises auf die Patenterteilung: **25.06.2014**
(21) Anmeldenummer: 11158245.8
(22) Anmeldetag: 18.12.2007
(51) Int. Cl.: C12N 15/45, C12N 15/85, C12N 15/861, C07K 14/135, A61K 39/155

(54) **RSV F-Protein und dessen Verwendung**
RSV F protein and its use
Protéine RSV F et son utilisation

(30) Priorität: 21.12.2006 DE 102006060799
(43) Veröffentlichungstag der Anmeldung: 28.09.2011
(62) Teilanmeldung aus: 07856822.7
(73) Patentinhaber: Ruhr-Universität Bochum, 44801 Bochum (DE)
(72) Erfinder: Grunwald, Thomas, 44791 Bochum (DE); Überla, Klaus, 45549 Sprockhövel (DE)
(74) Vertreter: Viering, Jentschura & Partner Patent- und Rechtsanwälte

(56) Entgegenhaltungen:
- WO-A-00/18929
- PARK E.-K. ET AL.: "Immune induction and modulation in mice following immunization with DNA encoding F protein of respiratory syncytial virus", MOLECULS & CELLS, Bd. 12, Nr. 1, 2001, Seiten 50-56, XP002476630,
- LI XIAOMAO ET AL: "Protection against respiratory syncytial virus infection by DNA immunization", JOURNAL OF EXPERIMENTAL MEDICINE, TOKYO, JP, Bd. 188, Nr. 4, 17. August 1998 (1998-08-17), Seiten 681-688, XP002133951, ISSN: 0022-1007
- TREE J A ET AL: "An assessment of different DNA delivery systems for protection against respiratory syncytial virus infection in the murine model: gene-gun delivery induces IgG in the lung", VACCINE, BUTTERWORTH SCIENTIFIC. GUILDFORD, GB, Bd. 22, Nr. 19, 23. Juni 2004 (2004-06-23) , Seiten 2438-2443, XP004515462, ISSN: 0264-410X

### SEQUENCE LISTING

<110> Ruhr- Uni ver si t ät Bochum
<120> RSV-F exprimierende Nukl eot idsequenzen und ihre Verwendung
<130> P46341
<160> 5
<170> Patent I nversion 3.3
<210> 1
   <211> 1725
   <212> DNA
   <213> respiratory syncytial virus
<400> 1
   atggagttgc caatcctcaa agcaaatgca attaccacaa tcctcgctgc agtcacattt     60
   tgctttgctt ctagtcaaaa catcactgaa gaattttatc aatcaacatg cagtgcagtt    120
   agcaaaggct atcttagtgc tctaagaact ggttggtata ctagtgttat aactatagaa    180
   ttaagtaata tcaaggaaaa taagtgtaat ggaacagatg ctaaggtaaa attgatgaaa    240
   caagaattag ataaatataa aaatgctgta acagaattgc agttgctcat gcaaagcaca    300
   ccagcagcaa acaatcgagc cagaagagaa ctaccaaggt ttatgaatta tacactcaac    360
   aataccaaaa aaaccaatgt aacattaagc aagaaaagga aaagaagatt tcttggtttt    420
   ttgttaggtg ttggatctgc aatcgccagt ggcattgctg tatctaaggt cctgcactta    480
   gaaggagaag tgaacaagat caaaagtgct ctactatcca caaacaaggc cgtagtcagc    540
   ttatcaaatg gagttagtgt cttaaccagc aaagtgttag acctcaaaaa ctatatagat    600
   aaacaattgt tacctattgt gaataagcaa agctgcagaa tatcaaatat agaaactgtg    660
   atagagttcc aacaaaagaa caacagacta ctagagatta ccagggaatt tagtgttaat    720
   gtaggtgtaa ctacacctgt aagcacttac atgttaacta atagtgaatt attgtcatta    780
   atcaatgata tgcctataac aaatgatcag aaaaagttaa tgtccaacaa tgttcaaata    840
   gttagacagc aaagttactc tatcatgtcc ataataaaag aggaagtctt agcatatgta    900
   gtacaattac cactatatgg tgtgatagat acaccttgtt ggaaattaca cacatcccct    960
   ctatgtacaa ccaacacaaa agaagggtca aacatctgtt taacaagaac tgacagagga   1020
   tggtactgtg acaatgcagg atcagtatct ttcttcccac aagctgaaac atgtaaagtt   1080
   caatcgaatc gagtattttg tgacacaatg aacagtttaa cattaccaag tgaagtaaat   1140
   ctctgcaatg ttgacatatt caatcccaaa tatgattgta aaattatgac ttcaaaaaca   1200
   gatgtaagca gctccgttat cacatctcta ggagccattg tgtcatgcta tggcaaaact   1260
   aaatgtacag catccaataa aaatcgtgga atcataaaga cattttctaa cgggtgtgat   1320
   tatgtatcaa ataaaggggt ggacactgtg tctgtaggta acacattata ttatgtaaat   1380
   aagcaagaag gcaaaagtct ctatgtaaaa ggtgaaccaa taataaattt ctatgaccca   1440
   ttagtattcc cctctgatga atttgatgca tcaatatctc aagtcaatga gaagattaac   1500
   cagagtttag catttattcg taaatccgat gaattattac ataatgtaaa tgctggtaaa   1560
   tcaaccacaa atatcatgat aactactata attatagtga ttatagtaat attgttatca   1620
   ttaattgctg ttggactgct cctatactgt aaggccagaa gcacaccagt cacactaagc   1680
   aaggatcaac tgagtggtat aaataatatt gcatttagta actaa                   1725
<210> 2
   <211> 1728
   <212> DNA
   <213> Artificial
<220>
   <223> Für di e Expr essi on in humanen Wirtszellen codon-optimier t e, das F Protein von RSVkodi er ende Nukleotidsequenz
<400> 2
   atggagctgc ctatcctgaa ggccaacgcc atcaccacaa ttctggccgc cgtgaccttc     60
   tgttttgcca gcagccagaa catcaccgag gagttctacc agagcacctg tagcgccgtg    120
   agcaagggct atctgagcgc cctgagaacc ggctggtaca ccagcgtgat caccatcgag    180
   ctgagcaaca tcaaggagaa caagtgcaac ggcaccgacg ccaaggtgaa gctgatgaag    240
   caggagct gg  acaagt acaa  gaacgccgt g  accgaact gc  agct gct gat  gcagt ct acc   300
   cctgccgcca acaacagagc cagacgggag ctgccccggt tcatgaacta caccctgaac    360
   aacaccaaga aaaccaacgt gaccctgagc aagaagcgga agcggagatt cctgggcttt    420
   ct gct gggag  t gggct ct gc  cat cgcct ct  ggcat cgccg  t gt ct aaggt  gct gcacct g   480
   gagggagagg tgaacaagat caagagcgcc ctgctgagca ccaataaggc cgtggtgagc    540
   ct gagcaat g  gcgt gagcgt  gct gacaagc  aaggt gct gg  acct caagaa  ct acat cgac   600
   aagcagctgc tgcccatcgt gaacaagcag agctgccgga tcagcaacat cgagaccgtg    660
   atcgagttcc agcagaagaa caaccggctg ctggagatca ccagggagtt cagcgtgaat    720
   gtgggcgtga ccacccctgt gagcacctac atgctgacca acagcgagct gctgagcctg    780
   atcaacgaca tgcccatcac caacgaccag aagaagctga tgtccaacaa cgtgcagatc    840
   gt gcggcagc  agagct acag  cat cat gt cc  at cat caagg  aggaggt gct  ggct t acgt g   900
   gtgcagctgc ctctgtacgg cgtgatcgac accccttgct ggaagctgca caccagccct    960
   ctgtgcacca ccaataccaa ggagggcagc aacatctgcc tgaccaggac cgatagaggc   1020
   tggtactgcg acaatgccgg cagcgtgagc ttctttccac aggccgagac ctgtaaggtg   1080
   cagagcaacc gggtgttctg cgacaccatg aacagcctga ccctgccttc tgaggtgaac   1140
   ctgtgcaacg tggacatctt caaccccaag tacgactgca agatcatgac cagcaagacc   1200
   gacgtgagca gcagcgtgat tacaagcctg ggcgccatcg tgagctgtta cggcaagacc   1260
   aagtgcaccg ccagcaacaa gaaccgcggc atcatcaaga ccttcagcaa cggctgcgac   1320
   tacgtgagca acaagggcgt ggatacagtg agcgtgggca acaccctgta ctacgtcaac   1380
   aagcaggagg gcaagagcct gtacgtgaag ggcgagccca tcatcaactt ctacgacccc   1440
   ct ggt gt t cc  ct agcgacga  gt t cgat gcc  agcat cagcc  aggt gaacga  gaagat caac  1500
   cagagcctgg ccttcatcag gaagagcgac gagctgctgc acaatgtgaa cgccggcaag   1560
   agcaccacca acatcatgat caccaccatc atcatcgtga tcatcgtcat cctgctgtcc   1620
   ctgattgctg tgggcctgct gctgtactgt aaggccagaa gcacccccgt gaccctgtct   1680
   aaggat cagc  t gagcggcat  caacaacat c  gcct t ct cca  act gat aa                 1728
<210> 3
   <211> 574
   <212> PRT
   <213> respiratory syncytial virus
<400> 3
   Met Glu Leu Pro Ile Leu Lys Ala Asn Ala Ile Thr Thr Ile Leu Ala
   1 5 10 15
   Ala Val Thr Phe Cys Phe Ala Ser Ser Gln Asn Ile Thr Glu Glu Phe
   20 25 30
   Tyr Gln Ser Thr Cys Ser Ala Val Ser Lys Gly Tyr Leu Ser Ala Leu
   35 40 45
   Arg Thr Gly Trp Tyr Thr Ser Val Ile Thr Ile Glu Leu Ser Asn Ile
   50 55 60
   Lys Glu Asn Lys Cys Asn Gly Thr Asp Ala Lys Val Lys Leu Met Lys
   65 70 75 80
   Gln Glu Leu Asp Lys Tyr Lys Asn Ala Val Thr Glu Leu Gln Leu Leu
   85 90 95
   Met Gln Ser Thr Pro Ala Ala Asn Asn Arg Ala Arg Arg Glu Leu Pro
   100 105 110
   Arg Phe Met Asn Tyr Thr Leu Asn Asn Thr Lys Lys Thr Asn Val Thr
   115 120 125
   Leu Ser Lys Lys Arg Lys Arg Arg Phe Leu Gly Phe Leu Leu Gly Val
   130 135 140
   Gly Ser Ala Ile Ala Ser Gly Ile Ala Val Ser Lys Val Leu His Leu
   145 150 155 160
   Glu Gly Glu Val Asn Lys Ile Lys Ser Ala Leu Leu Ser Thr Asn Lys
   165 170 175
   Ala Val Val Ser Leu Ser Asn Gly Val Ser Val Leu Thr Ser Lys Val
   180 185 190
   Leu Asp Leu Lys Asn Tyr Ile Asp Lys Gln Leu Leu Pro Ile Val Asn
   195 200 205
   Lys Gln Ser Cys Arg Ile Ser Asn Ile Glu Thr Val Ile Glu Phe Gln
   210 215 220
   Gln Lys Asn Asn Arg Leu Leu Glu Ile Thr Arg Glu Phe Ser Val Asn
   225 230 235 240
   Val Gly Val Thr Thr Pro Val Ser Thr Tyr Met Leu Thr Asn Ser Glu
   245 250 255
   Leu Leu Ser Leu Ile Asn Asp Met Pro Ile Thr Asn Asp Gln Lys Lys
   260 265 270
   Leu Met Ser Asn Asn Val Gln Ile Val Arg Gln Gln Ser Tyr Ser Ile
   275 280 285
   Met Ser Ile Ile Lys Glu Glu Val Leu Ala Tyr Val Val Gln Leu Pro
   290 295 300
   Leu Tyr Gly Val Ile Asp Thr Pro Cys Trp Lys Leu His Thr Ser Pro
   305 310 315 320
   Leu Cys Thr Thr Asn Thr Lys Glu Gly Ser Asn Ile Cys Leu Thr Arg
   325 330 335
   Thr Asp Arg Gly Trp Tyr Cys Asp Asn Ala Gly Ser Val Ser Phe Phe
   340 345 350
   Pro Gln Ala Glu Thr Cys Lys Val Gln Ser Asn Arg Val Phe Cys Asp
   355 360 365
   Thr Met Asn Ser Leu Thr Leu Pro Ser Glu Val Asn Leu Cys Asn Val
   370 375 380
   Asp Ile Phe Asn Pro Lys Tyr Asp Cys Lys Ile Met Thr Ser Lys Thr
   385 390 395 400
   Asp Val Ser Ser Ser Val Ile Thr Ser Leu Gly Ala Ile Val Ser Cys
   405 410 415
   Tyr Gly Lys Thr Lys Cys Thr Ala Ser Asn Lys Asn Arg Gly Ile Ile
   420 425 430
   Lys Thr Phe Ser Asn Gly Cys Asp Tyr Val Ser Asn Lys Gly Val Asp
   435 440 445
   Thr Val Ser Val Gly Asn Thr Leu Tyr Tyr Val Asn Lys Gln Glu Gly
   450 455 460
   Lys Ser Leu Tyr Val Lys Gly Glu Pro Ile Ile Asn Phe Tyr Asp Pro
   465 470 475 480
   Leu Val Phe Pro Ser Asp Glu Phe Asp Ala Ser Ile Ser Gln Val Asn
                   485                 490                 495
   Glu Lys Ile Asn Gln Ser Leu Ala Phe Ile Arg Lys Ser Asp Glu Leu
               500                 505                 510
   Leu His Asn Val Asn Ala Gly Lys Ser Thr Thr Asn Ile Met Ile Thr
           515                 520                 525
   Thr Ile Ile Ile Val Ile Ile Val Ile Leu Leu Ser Leu Ile Ala Val
       530                 535                 540
   Gly Leu Leu Leu Tyr Cys Lys Ala Arg Ser Thr Pro Val Thr Leu Ser
   545                 550                 555                 560
   Lys Asp Gln Leu Ser Gly Ile Asn Asn Ile Ala Phe Ser Asn
                   565                 570
<210> 4
   <211> 37
   <212> DNA
   <213> Artificial
<220>
   <223> Oligonucleotide primer (sense)
<400> 4
   gat ccaagct t ccaccat gg agt t gccaat cct caaa 37
<210> 5
   <211> 41
   <212> DNA
   <213> Artificial
<220>
   <223> Oligonucleotide primer (antisense)
<400> 5
   t cgacct cga gt t agt t act aaat gcaat a tt attt at ac c 41

## Beschreibung

### Gebiet der Erfindung

Die vorliegende Erfindung richtet sich auf ein Nukleinsäuremolekül, das für das F-Protein des Respiratorischen Syncytialvirus oder ein Fragment davon kodiert, für die Expression in einer menschlichen Zellumgebung codon-optimierte Varianten dieses Nukleinsäuremoleküls und deren Verwendung mit viralen Vektoren oder PlasmidVektoren als Vakzine.

### Hintergrund der Erfindung

Bereits 1957 wurde bei Kindern mit schweren Erkrankungen der unteren Luftwege ein Virus nachgewiesen, das als Respiratorisches Syncytialvirus (RSV) bezeichnet wurde. Dieser Name weist auf die Eigenschaft des Virus hin, Erkrankungen des Respirationstrakts zu erzeugen und in vitro Syncytienbildung zu induzieren.

RSV gehört zur Familie der Paramyxoviren und darin zur Unterfamilie der Pneumovirinae. RSV hat wie die anderen Vertreter dieser Familie ein nicht-segmentiertes, durchgehendes RNA-Genom in Negativstrangorientierung. Das Genom des RSV ist 15222 Basen lang und liegt im Komplex mit Proteinen als Nucleocapsid vor.

Das Virusgenom kodiert eine Reihe von Virusproteinen. Darunter sind die Membranproteine, die als RSV G-Protein und RSV F-Protein bezeichnet werden. Das G-Protein ist für die spezifische Adsorption des Viruspartikels an die Zelloberfläche verantwortlich, während das F-Protein die Fusion der viralen mit der zellulären Membran induziert. Das F-Protein wird als Vorläuferpolypeptid F₀ synthetisiert und besitzt am N-terminalen Ende ein Signalpeptid für den Transport des Translationskomplexes zur Membran des endoplasmatischen Reticulums. Nach dem Durchschleusen der Aminosäurekette durch die Membran, bewirkt eine hydrophobe Sequenz am C-terminalen Ende die Verankerung des F₀ Proteins in der Membran und das Signalpeptid wird abgespalten. In der Folge wird das Protein während des Transports durch den Golgi-Apparat glykosyliert. Ebenfalls im Golgi-Apparat erfolgt eine Spaltung des F₀ Proteins in den aminoterminalen F₂ Teil und das F₁ Protein. Die Spaltstelle liegt zwischen einem Abschnitt basischer Aminosäuren und einer hydrophoben Domäne. Diese hydrophobe Domäne von etwa 25 Aminosäuren Länge bildet nach der Spaltung den N-Terminus des F₁ Proteins und vermittelt die Verschmelzung der viralen mit der zellulären Membran nach der Absorption. Das F₂ Protein bleibt mit dem F₁ Protein über eine Disulfidbrücke verbunden.

Antikörper, die gegen dieses fusionsvermittelnde Peptid des F₁ Proteins gerichtet sind, können die Aufnahme des Virus in die Zelle verhindern und haben daher neutralisierende Wirkung.

Die Infektion mit dem RSV ist hochansteckend, in einem Milliliter Speichel finden sich bis zu 10⁶ infektiöse Viruspartikel. Es wird vorwiegend durch Tröpfchen und direkten Kontakt mit infizierten Personen übertragen. Vor allem Kinder infizieren sich während der Wintermonate.

Das RSV gilt als das infektiologische Hauptproblem des ersten Lebensjahres. Insbesondere gefährdet sind Säuglinge im Alter zwischen sechs Wochen und einem halben Jahr. Im Alter von vier Jahren besitzen 80 % der Kinder Antikörper gegen das Virus.

Reinfektionen mit leichten Erkrankungsformen treten aber auch in höherem Alter als Folge einer absinkenden Antikörperkonzentration auf. Insbesondere häufig sind Nosokomialinfektionen in Altersheimen, Kindergärten und -kliniken.

Die Inkubationszeit des RSV beträgt etwa 4-5 Tage. Die Erkrankung zeigt sich durch leichte bis schwere lebensbedrohende grippale Infekte mit Fieber und Schnupfen. Häufig kommt es auch zu Entzündungen des Rachens (Pharyngitis) und der Luftröhre (Tracheitis) sowie der Bronchien (Bronchitis).

Nach der Tröpfcheninfektion des oberen Respirationstrakts vermehrt sich das Virus in den Zellen der Schleimhaut und kann sich von dort aus innerhalb von ein bis zwei Tagen in die unteren Luftwege ausbreiten.

Ein Impfstoff gegen RSV ist zurzeit noch nicht bekannt. Durch Formalin abgetötete Viren waren wenig erfolgreich, da das F-Protein durch die chemische Behandlung zerstört wird und nur Antikörper gegen das G-Protein gebildet werden. Diese sind zwar virusneutralisierend, können aber die Ausbreitung des Virus durch Zellfusionen nicht verhindern. Eine passive Immunisierung durch Immunglobulingabe wird angewandt, ist jedoch mit hohen Kosten verbunden und eignet sich daher nicht zur prophylaktischen Impfung größerer Bevölkerungsgruppen.

WO 00/18929 offenbart in diesem Zusammenhang immunogene Fusions-Fragmente von RSV, die das F-Protein umfassen.

Es besteht daher weiterhin ein Bedarf für einen wirksamen Impfstoff gegen eine Infektion mit dem RSV.

### Zusammenfassung der Erfindung

Die vorliegende Erfindung richtet sich auf ein Nukleinsäuremolekül, das eine Nukleotidsequenz umfasst, die für das F-Protein des Respiratorischen Syncytialvirus (RSV) oder ein immunogenes Fragment davon kodiert, sowie von diesem Nukleinsäuremolekül abgeleitete Polynukleotide, die für eine effiziente Expression des F-Proteins des RSV in einer Wirtszelle codon-optimiert wurden. Das Nukleinsäuremolekül umfasst die Nukleotidsequenz von einem Fragment von SEQ ID NO: 2 SEQ ID NO:2 ist eine für die Expression des F-Proteins in einer menschlichen Wirtszelle codon-optimierte Nukleotidsequenz.

Weiter beschrieben sind die durch die Expression der erfindungsgemäßen Nukleinsäuremoleküle erhaltenen Polypeptidmoleküle. Diese umfassen oder bestehen aus der Aminosäuresequenz mit SEQ ID NO: 3 oder einem Fragment davon.

Weiterhin umfasst die vorliegende Erfindung auch einen Vektor, der eins der erfindungsgemäßen Nukleinsäuremoleküle beinhaltet. Die erfindungsgemäßen Nukleinsäuremoleküle können in dem Vektor in Form einer (Expressions)-Kassette enthalten sein, die neben der erfindungsgemäßen Nukleotidsequenz auch eine mit der Nukleotidsequenz funktional verknüpfte Transkriptions- und/oder Translationskontrollsequenz, wie beispielsweise einen Promotor, umfassen kann. Noch ein weiterer Aspekt dieser Erfindung sind Zellen, die einen solchen Vektor enthalten.

In einer bevorzugten Ausführungsform der Erfindung ist der verwendete Vektor ein viraler Vektor oder ein Plasmid. Besonders bevorzugt ist ein adenoviraler Vektor, in welchem die E1-Region zumindest teilweise deletiert wurde, so dass der adenovirale Vektor replikationsdefizient ist. Zusätzlich kann gegebenenfalls auch die E3-Region deletiert sein.

In einer Ausführungsform der Erfindung enthält der adenovirale Vektor die erfindungsgemäße Nukleotidsequenz in Form einer Expressionskassette, in der die das F Protein des RSV kodierende Nukleotidsequenz funktional mit einem geeigneten Promotor, beispielsweise dem CMV Promotor, verknüpft ist. Der verwendete Promotor im adenoviralen Vektor ist vorzugsweise regulierbar, beispielsweise ein Tetrazyklin-regulierbarer Promotor, um in der adenoviralen Produktionszelle die Expression des F-Proteins von RSV zu verhindern. In allen Zellen, die das Regulationssystem nicht enthalten, ist der Promotor aktiv und exprimiert das F-Protein von RSV. Ein solcher adenoviraler Vektor kann als Vakzin-Vektor eingesetzt werden

In einem weiteren Aspekt, umfasst die vorliegende Erfindung auch Plasmidvektoren, die die erfindungsgemäßen Nukleotidsequenzen umfassen. In einer Ausführungsform können diese Plasmidvektoren als Shuttle Vektoren eingesetzt werden und umfassen daher einen Nukleinsäureanteil der die homologe Rekombination mit einem geeigneten Rückgratplasmid, das beispielsweise ein Großteil eines viralen Genoms enthält, ermöglicht. Besonders bevorzugt sind im Shuttle Vektor adenovirale Sequenzen enthalten, die die homologe Rekombination mit einem anderen Plasmid, das den Großteil des adenoviralen Genoms enthält, erlauben. Auf diesem Weg lassen sich beispielsweise die oben genannten adenoviralen Vakzin-Vektoren herstellen.

Weiterhin bezieht sich die vorliegenden Erfindung in einem weiteren Aspekt auf immunogene Zusammensetzungen, die die erfindungsgemäßen Nukleotidsequenzen mit Fragmenten von SEQ ID NO: 2 umfassen. In einer solchen Zusammensetzung kann die erfindungsgemäße Nukleotidsequenz auch in Form der oben erwähnten Vektoren, vorzugsweise in Form eines adenoviralen Vakzin-Vektors, vorliegen. Ein solcher Vektor erlaubt den Transport der erfindungsgemäßen Nukleinsäuremoleküle in einen menschlichen Wirt und die Expression des kodierten Proteins. Das Protein wird dabei in einer Menge exprimiert, die ausreicht die beabsichtigte Immunantwort auszulösen.

Die immunogenen Zusammensetzungen können abhängig von der gewünschten Art der Anwendung auch einen pharmazeutisch annehmbaren Träger und gegebenenfalls weitere Hilfsstoffe enthalten.

Ein weiterer Aspekt ist die Verwendung der erfindungsgemäßen Nukleinsäuremoleküle oder Vektoren für die Herstellung einer Impfstoffzusammensetzung für die Impfung eines Subjekts gegen durch die Infektion mit RSV hervorgerufene Erkrankungen. Dieses Subjekt ist vorzugsweise ein Mensch.

Die vorliegende Erfindung bezieht sich außerdem auf ein Verfahren zur Herstellung des RSV F-Proteins, wobei das Verfahren die Expression eines erfindungsgemäßen Nukleinsäuremoleküls in einer geeigneten Wirtszelle umfasst. In einer bevorzugten Ausführungsform dieses Verfahrens ist das erfindungsgemäße Nukleinsäuremolekül für die Expression in der Wirtszelle codon-optimiert.

### Kurze Beschreibung der Zeichnungen

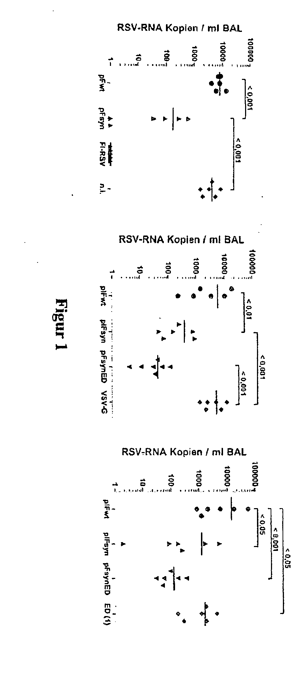

**Figur 1** zeigt die Ergebnisse einer RSV-qRT-PCR von RNA-Isolaten aus einer Bronchoalveolären Lavage (BAL) von Mäusen die mit unterschiedlichen Plasmid-DNA-Konstrukten immunisiert wurden. Die Zahlen über den Klammern geben die statistische Signifikanz des Unterschieds zwischen den Einzelergebnissen der verglichenen Gruppen an (Tukey-Test).

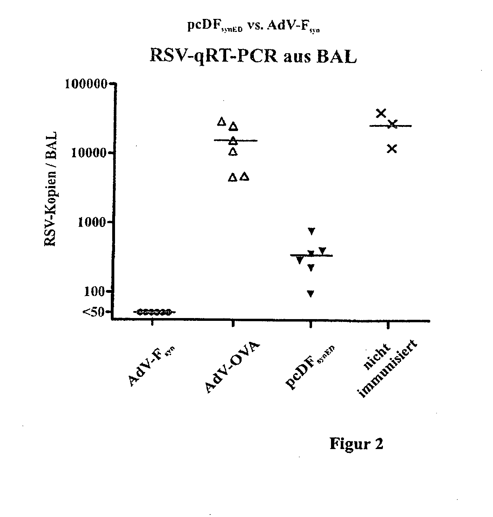

**Figur 2** zeigt die Ergebnisse einer RSV-qRT-PCR von RNA-Isolaten aus einer Bronchoalveolären Lavage (BAL) von Mäusen die mit unterschiedlichen Konstrukten immunisiert wurden nach Infektion mit RSV. Die Kopienzahl von RSV nach qRT-PCR wurde durch RNA-Quantifizierung standardisiert und ist hier für die RNA-Isolate der gewonnenen BALs dargestellt. Gezeigt sind jeweils die Einzelwerte von 6 Mäusen (schwarze Symbole) sowie der Mittelwert (Balken).

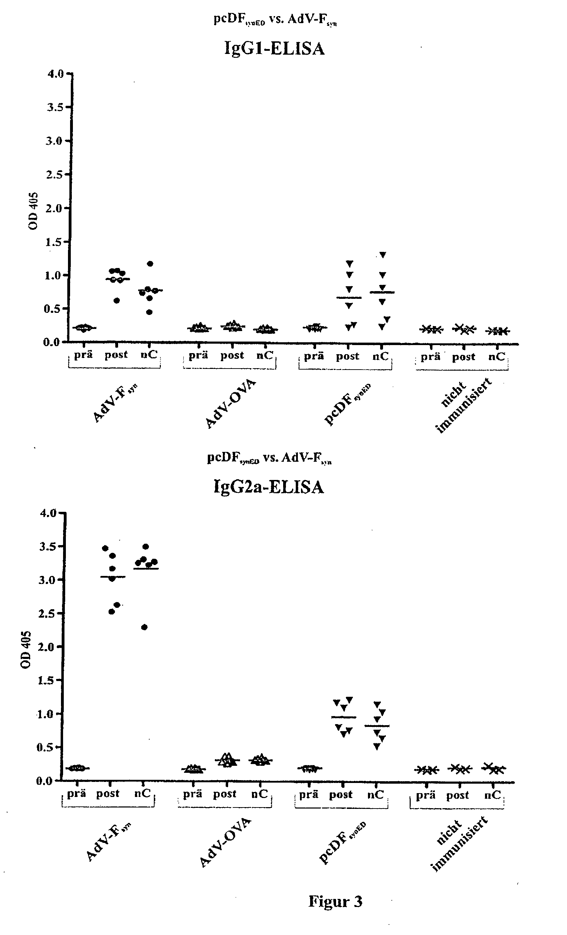

**Figur 3** zeigt die Ergebnisse eines IgG-Antikörper-ELISAs. Die vor der ersten Immunisierung (prä), nach der zweiten Immunisierung (post) und nach dem Challenge (ca. 1 x 10⁷ iE (infektiöse Einheiten) von Plaque aufgereinigtem RSV intranasal) am Tötungstag (nC) gewonnenen Serumproben der mit unterschiedlichen Zusammensetzungen (AdV-F_{syn}: für das synthetische (codon-optimierte) F-Protein von RSV kodierende Nukleinsäure in adenoviralem Vektor; AdV-Ova: adenoviraler Vektor mit Nukleinsäure, die für Ovalbumin kodiert; pcDF_{syn ED}: Plasmid, das für die Ektodomäne des synthetischen (codon-optimierten) F-Proteins von RSV kodiert) immunisierten bzw. nicht immunisierten Mäuse wurden im IgG-Antikörper-ELISA auf RSV-spezifische IgG1- und IgG2a-Antikörper getestet. Die Stärke der Absorption bei der Wellenlänge 405 entspricht dem Antikörpertiter im Blut der Mäuse. Gezeigt sind jeweils die Einzelwerte von 6 Mäusen (schwarze Symbole) sowie der Mittelwert (Balken).

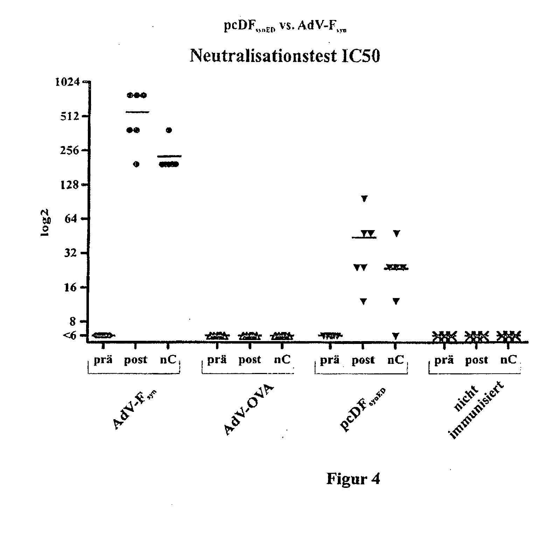

**Figur 4** zeigt das Ergebnis eines Neutralisationstests. Die vor der ersten Immunisierung (prä), nach der zweiten Immunisierung (post) und nach der Challenge am Tötungstag (nC) gewonnenen Serumproben der Mäuse wurden seriell verdünnt und dann die Verdünnungsreihe im Neutralisationstest auf neutralisierende Antikörper gegen RSV getestet. Dargestellt ist jeweils die höchste Serumverdünnung, bei der die Infektion durch RSV durch neutralisierende Antikörper zu 50% (IC50) gehemmt wird. Gezeigt sind jeweils die Einzelwerte von 6 Mäusen (schwarze Symbole) sowie der Mittelwert (Balken).

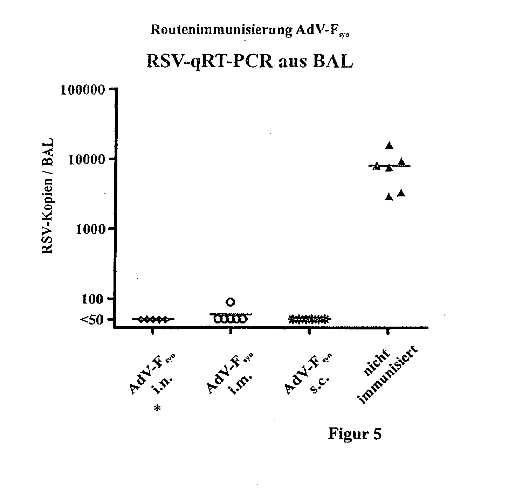

**Figur 5** zeigt eine RSV-qRT-PCR von RNA-Isolaten aus einer Brocho-alveolären Lavage (BAL) von Mäusen, die über unterschiedliche Verabreichungswege mit einem erfindungsgemäßen adenoviralen Vektor, der ein für ein codon-optimiertes RSV-F Protein kodierendes Nukleinsäuremolekül umfasst, immunisiert wurden. Die Kopienzahl von RSV nach qRT-PCR wurde durch RNA-Quantifizierung standardisiert und ist hier für die RNA-Isolate der am Tötungstag gewonnenen BALs dargestellt. Gezeigt sind jeweils die Einzelwerte von 6 Mäusen (schwarze Symbole) sowie der Mittelwert (Balken).

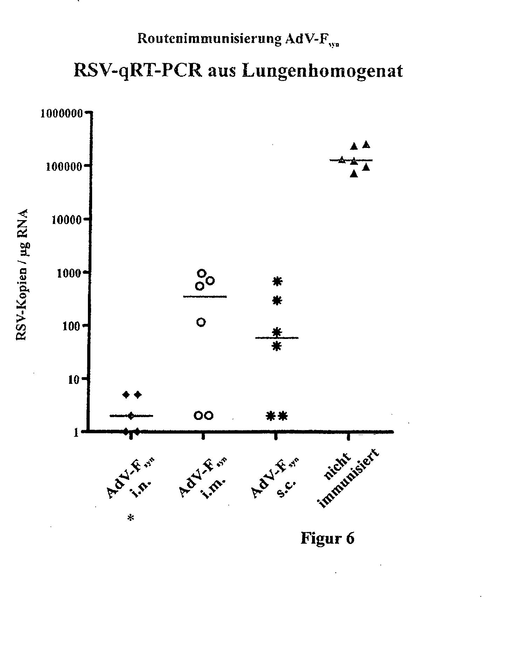

**Figur 6** zeigt eine RSV-qRT-PCR von RNA-Isoläten aus dem Lungenhomogenat von Mäusen, die über unterschiedliche Verabreichungswege mit einem erfindungsgemäßen adenoviralen Vektor, der ein für ein codon-optimiertes RSV-F Protein kodierendes Nukleinsäuremolekül umfasst, immunisiert wurden. Es sind die Ergebnisse der qRT-PCR von den RNA-Isolaten der am Tötungstag gewonnenen Lungenhomogenate dargestellt. Die Kopienzahl von RSV nach qRT-PCR wurde durch RNA-Quantifizierung standardisiert und in Bezug zum RNA-Gehalt der Isolate umgerechnet. Gezeigt sind jeweils die Einzelwerte von 5 (*) oder 6 Mäusen (schwarze Symbole) sowie der Mittelwert (Balken).

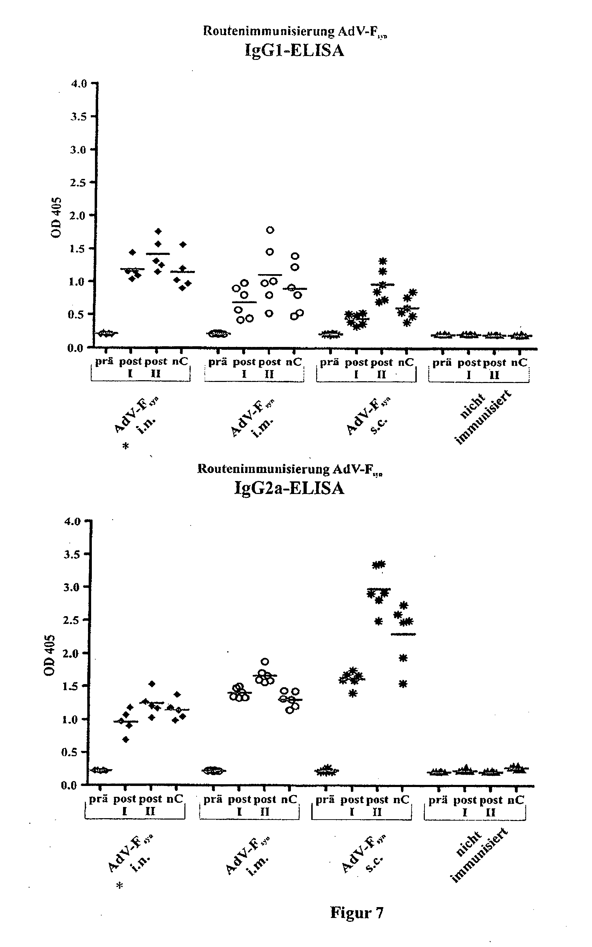

**Figur 7** zeigt einen IgG-Antikörper-ELISA von Mäusen, die über unterschiedliche Verabreichungswege mit einem erfindungsgemäßen adenoviralen Vektor, der ein für ein codon-optimiertes RSV-F Protein kodierendes Nukleinsäuremolekül umfasst, immunisiert wurden. Die vor der ersten Immunisierung (prä), nach der ersten Immunisierung (post I), nach der zweiten Immunisierung (post II) und nach dem Challenge am Tötungstag (nC) gewonnenen Serumproben der Mäuse wurden im IgG-Antikörper-ELISA auf RSV-spezifische IgG1- und IgG2a-Antikörper getestet. Die Stärke der Absorption bei der Wellenlänge 405 entspricht dem Antikörpertiter im Blut der Mäuse. Gezeigt sind jeweils die Einzelwerte von 5 (*) oder 6 Mäusen (schwarze Symbole) sowie der Mittelwert (Balken).

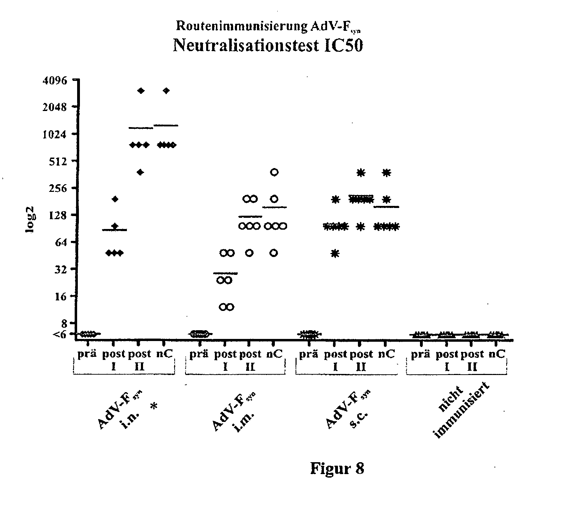

**Figur 8** zeigt das Ergebnis eines Neutralisationstests von Mäusen, die über unterschiedliche Verabreichungswege mit einem erfindungsgemäßen adenoviralen Vektor, der ein für ein codon-optimiertes RSV-F Protein kodierendes Nukleinsäuremolekül umfasst, immunisiert wurden. Die vor der ersten Immunisierung (prä), nach der ersten Immunisierung (post I), nach der zweiten Immunisierung (post II) und nach dem Challenge am Tötungstag (nC) gewonnenen Serumproben der Mäuse wurden seriell verdünnt und dann die Verdünnungsreihe im Neutralisationstest auf neutralisierende Antikörper gegen RSV getestet. Dargestellt ist jeweils die höchste Serumverdünnung, bei der die Infektion durch RSV durch neutralisierende Antikörper zu 50% (IC50) gehemmt wird. Gezeigt sind jeweils die Einzelwerte von 5 (*) oder 6 Mäusen (schwarze Symbole) sowie der Mittelwert (Balken).

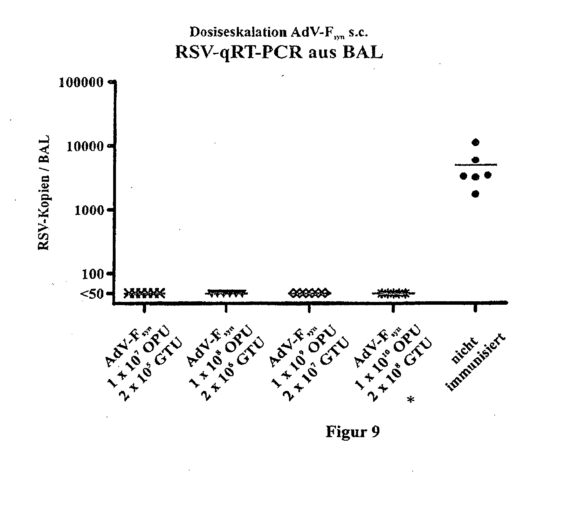

**Figur 9** zeigt die Ergebnisse einer RSV-qRT-PCR von RNA-Isolaten aus der Bronchoalveolären Lavage (BAL) von Mäusen nach subkutaner Immunisierung mit einem erfindungsgemäßen adenoviralen Vektor, der ein für ein codon-optimiertes RSV-F Protein kodierendes Nukleinsäuremolekül umfasst (AdV-F_{syn}), in verschiedenen Dosierungen (Dosiseskalation). Die Kopienzahl von RSV nach qRT-PCR wurde durch RNA-Quantifizierung standardisiert und ist für die RNA-Isolate der am Tötungstag gewonnenen BALs dargestellt. Gezeigt sind jeweils die Einzelwerte von 5 (*) oder 6 Mäusen (schwarze Symbole) sowie der Mittelwert (Balken).

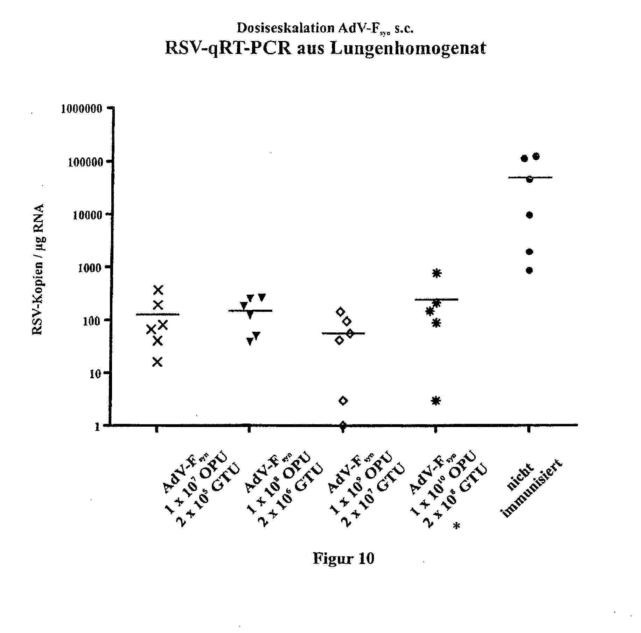

**Figur 10** zeigt die Ergebnisse einer RSV-qRT-PCR von RNA-Isolaten aus dem Lungenhomogenat von Mäusen nach subkutaner Immunisierung mit einem erfindungsgemäßen adenoviralen Vektor, der ein für ein codon-optimiertes RSV-F Protein kodierendes Nukleinsäuremolekül umfasst (AdV-F_{syn}), in verschiedenen Dosierungen (Dosiseskalation). Es sind die Ergebnisse der qRT-PCR von den RNA-Isolaten der am Tötungstag gewonnenen Lungenhomogenate dargestellt. Die RSV-Kopienzahl nach qRT-PCR wurde durch RNA-Quantifizierung standardisiert und in Bezug zum RNA-Gehalt der Isolate umgerechnet. Gezeigt sind jeweils die Einzelwerte von 5 (*) oder 6 Mäusen (schwarze Symbole) sowie der Mittelwert (Balken).

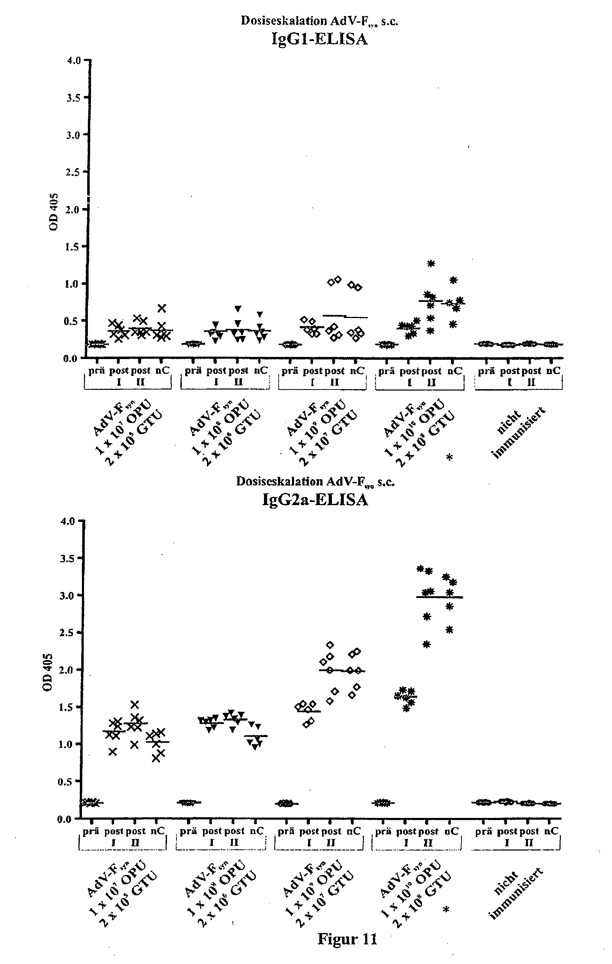

**Figur 11** zeigt das Ergebnis eines IgG-Antikörper-ELISAs von Mäusen nach subkutaner Immunisierung mit einem erfindungsgemäßen adenoviralen Vektor, der ein für ein codon-optimiertes RSV-F Protein kodierendes Nukleinsäuremolekül umfasst (AdV-F_{syn}), in verschiedenen Dosierungen (Dosiseskalation). Die vor der ersten Immunisierung (prä), nach der ersten Immunisierung (post I), nach der zweiten Immunisierung (post II) und nach dem Challenge am Tötungstag (nC) gewonnenen Serumproben der Mäuse wurden im IgG-Antikörper-ELISA auf RSV-spezifische IgG1- und IgG2a-Antikörper getestet. Die Stärke der Absorption bei der Wellenlänge 405 entspricht dem Antikörpertiter im Blut der Mäuse. Gezeigt sind jeweils die Einzelwerte von 5 (*) oder 6 Mäusen (schwarze Symbole) sowie der Mittelwert (Balken).

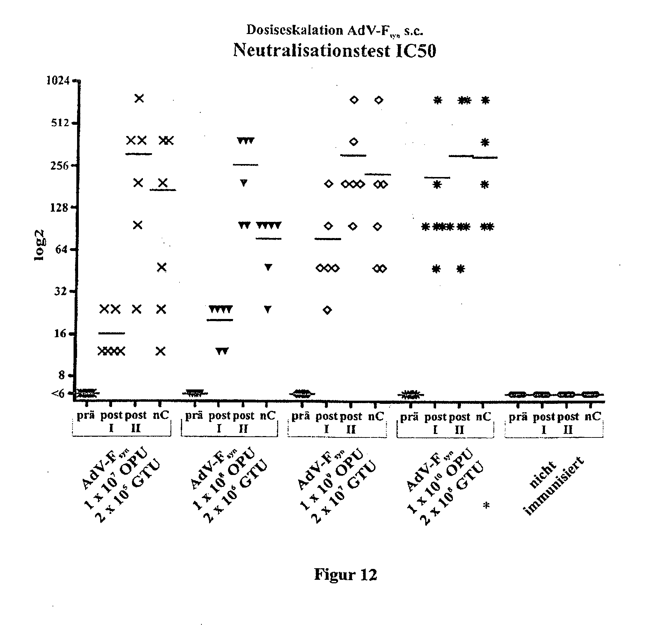

**Figur 12** zeigt das Ergebnis eines Neutralisationstests von Mäusen nach subkutaner Immunisierung mit einem erfindungsgemäßen adenoviralen Vektor, der ein für ein codon-optimiertes RSV-F Protein kodierendes Nukleinsäuremolekül umfasst (AdV-F_{syn}), in verschiedenen Dosierungen (Dosiseskalation). Die vor der ersten Immunisierung (prä), nach der ersten Immunisierung (post I), nach der zweiten Immunisierung (post II) und nach dem Challenge am Tötungstag (nC) gewonnenen Serumproben der Mäuse wurden seriell verdünnt und dann die Verdünnungsreihe im Neutralisationstest auf neutralisierende Antikörper gegen RSV getestet. Dargestellt ist jeweils die höchste Serumverdünnung, bei der die Infektion durch RSV durch neutralisierende Antikörper zu 50% (IC50) gehemmt wird. Gezeigt sind jeweils die Einzelwerte von 5 (*) oder 6 Mäusen (schwarze Symbole) sowie der Mittelwert (Balken).

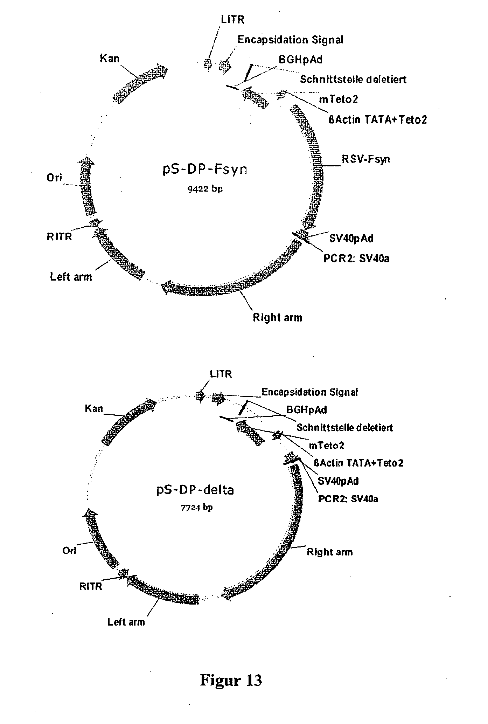

**Figur 13** zeigt die Vektorkarte eines Shuttleplasmids, in das über die Restriktionsstellen HindIII und XhoI das codon-optimierte RSV-F einkloniert wurde (pS-DP-Fsyn), bzw. eines Shuttleplasmids, das zur Aufnahme von codon-optimierten RSV-F verwendet wurde, und das einen Tetrazyklin-abhängigen Promotor enthält (pS-DP-delta). Dieser Promotor wird über ein genetisches Schalterelement in 293TRex-Zellen (Invitrogen) ausgeschaltet.

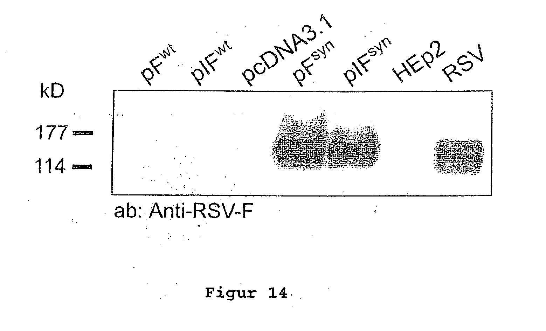

**Figur 14** zeigt Expressionsunterschiede von pFwt (Expressionsplasmid mit der Wildtyp Sequenz des RSV-F Proteins), pIFwt (Expressionsplasmid mit der Wildtyp Sequenz des RSV-F Proteins mit einem zusätzlichen Intron vor dem offenen Leserahmen), pFsyn (Expressionsplasmid mit der codon-optimierten Sequenz des RSV-F Proteins) und pIFsyn (Expressionsplasmid mit der codon-optimierten Sequenz des RSV-F Proteins mit einem zusätzlichen Intron vor dem offenen Leserahmen) nach Transfektion in Hep2 Zellen, Lyse der Zellen 48 Stunden nach der Transfektion, Auftrennung der Proteine des Zelllysats mittels Gelelektrophorese, Transfer auf eine Nitrocellulosemembran und Detektion mittels eines spezifischen Antikörpers (Ab: anti-RSV-F). pcDNA3.1: Plasmid ohne Fremdgen (Leerplasmid); Hep2: Zellen ohne Plasmid; RSV: mit RSV infizierte Zellen (Positivkontrolle); kD: Molekulargewicht in Kilodalton.

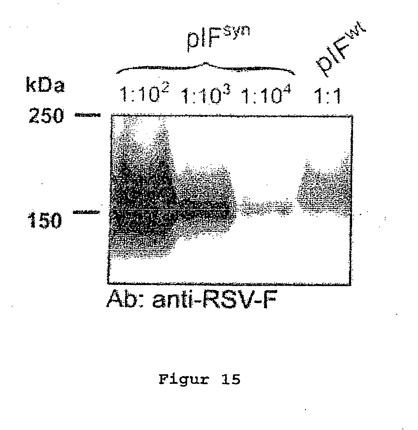

**Figur 15** zeigt eine Analyse der Expressionsstärken. Die Expression des codon-optimierten Konstrukts des RSV-F Proteins mit vorgeschaltetem Intron (pIFsyn) wurde durch serielle Verdünnung (1:10²bis 1:10⁴) des Zelllysates mit der Expression des Plasmids mit der Wildtyp Sequenz mit vorgeschaltetem Intron (pIFwt) im unverdünnten Zelllysat (1:1) verglichen. Die Proteinmenge von RSV-F durch Expression des codon-optimierten Konstrukts liegt bei einer Verdünnung von 1000-fach (1:10³) noch deutlich über der durch das Plasmid mit der ursprünglichen Sequenz. (kDa = Molekulargewicht in Kilodalton).

### Detaillierte Beschreibung der Erfindung

Gegenstand der vorliegenden Erfindung ist in einem ersten Aspekt ein Nukleinsäuremolekül, das eine Nukleotidsequenz umfasst, die für das F Protein des Respiratorischen Syncytialvirus (RSV) oder ein Fragment davon kodiert, wobei diese Nukleotidsequenz Fragmente von SEQ ID NO: 2 umfasst. Diese Sequenz unterscheidet sich von den bekannten das F Protein von RSV kodierenden Sequenzen unter anderem durch eine Substitution eines Nukleotids in der kodierenden Sequenz. Diese Substitution führt zu einer geänderten Aminosäuresequenz des F Proteins von RSV, in der sich an Position 241 ein Valin anstelle eines Alanins befindet. Dieses Nukleinsäuremolekül kann als Impfstoff oder Teil einer Impfstoffzusammensetzung verwendet werden.

"Fragment" im Zusammenhang mit einer Nukleinsäure bezieht sich auf Teile einer Nukleinsäuresequenz, die gegenüber dieser 3'- und/oder 5'-verkürzt sind. Insbesondere sind solche Fragmente mindestens 100 Nukleotide lang und kodieren einen Teil eines RSV F Proteins, der eine ausreichende Immunogenität besitzt, um in einem Organismus eine Immunantwort auszulösen.

"Immunogen" oder "Immunogenität" bezieht sich auf die Fähigkeit einer Substanz, beispielsweise eines Peptids oder Proteins, in einem Organismus eine Immunantwort hervorzurufen. Die Immunogenität einer Substanz kann beispielsweise über den Nachweis von Antikörpern bestimmt werden.

Das Nukleinsäuremolekül ist ein abgeleitetes synthetisches Nukleinsäuremolekül, das für die Expression in einem Wirtsorganismus, einem Wirtsgewebe oder einer Wirtszelle codon-optimiert wurde.

"Synthetisch" im Zusammenhang mit einer Nukleinsäure bedeutet im Kontext der vorliegenden Erfindung, dass es sich um ein Nukleinsäuremolekül handelt, das nicht natürlich vorkommt. Ein solches synthetisches Nukleinsäuremolekül kann beispielsweise ein codon-optimiertes Nukleinsäuremolekül sein.

"Codon-optimiert" im Zusammenhang mit einem Nukleinsäuremolekül bezieht sich auf ein Nukleinsäuremolekül, dessen Nukleotidsequenz so verändert worden ist, dass die Expression in einem Wirtsorganismus verbessert wird, d.h. eine größere Menge des durch das Nukleinsäuremolekül kodierten Proteins hergestellt wird.

In der Vergangenheit waren Versuche das F Protein des Respiratorischen Syncytial Virus (RSV) durch Transfektion mit Polymerase II abhängigen DNA Expressionsplasmiden zu exprimieren nicht erfolgreich.

Eukaryotische Zellen unterscheiden sich von prokaryoptischen Zellen durch eine stärkere Kompartimentalisierung des Intrazellularraums, was dazu dient komplexe enzymatische Reaktion, die für eine effiziente Proteinexpression, den Zellmetabolismus und/oder die Zellteilung notwendig sind, zu ermöglichen. In die Anpassung an die Wirtszelle und insbesondere an deren Expressionsmaschinerie ist der Schlüssel für die Replikation jedes Virus. RNA Viren, die sich im Cytoplasma ihrer Wirtszellen replizieren, haben sich unter den Umgebungsbedingungen des Cytoplasmas entwickelt. Diese Viren besitzen daher ihre eigene Transkriptionsmaschinerie, die eine RNA-abhängige RNA Polymerase, die zu der Synthese von mRNA aus der genomischen RNA führt, umfasst. Aus diesem Grund sind cytoplasmatische RNA Viren nicht an das komplexe nukleäre Milieu der eukaryotischen Wirtszelle angepasst. Die ineffiziente Expression von viralen Genen unter der Kontrolle eines eukaryotischen Promotors kann daher durch das Fehlen von Elementen, die für die Stabilisierung der prä-inRNA, die mRNA-Prozessierung und/oder den Export aus dem Kern erforderlich sind, erklärt werden. Das Ändern der Codons von viralen Genen zu den Codons, die am häufigsten von der Wirtszelle verwendet werden, erlaubt die wirksame Expression von viralen Genen.

Aus diesem Grund wurde die Nukleinsäuresequenz mit der SEQ ID NO: 1 in Übereinstimmung mit dieser Erfindung in eine synthetische, codon-optimierte Sequenz umgewandelt, die zu einer identischen translatierten Sequenz führt, aber alternative Codons verwendet. Das Verfahren der Codon-Optimierung umfasst dabei die folgenden Schritte: Identifizierung der Platzierung der Codons für einen exakten offenen Leserahmen, Vergleich der Codons mit der Verwendungshäufigkeit in dem gewünschten Wirtsorganismus, falls das Codon nicht das am häufigsten verwendete ist, Ersetzen durch das optimale Codon für die Expression, Wiederholen dieses Vorgangs für das gesamte Gensegment, Überprüfen der neuen Gensequenz auf unerwünschte Sequenzen, die durch den Codon-Austausch erzeugt wurden, wie beispielsweise Poly-Adenylierungsstellen, unerwünschte Restriktionsstellen, Intron-Splice-Erkennungsstellen etc., Zusammensetzen der synthetischen Gensegmente und Testen der Expression in einem Wirtsorganismus oder einer Wirtszelle.

Ein Beispiel für eine mittels dieses Verfahrens erhaltene für die Expression in menschlichen Zellen codon-optimierte, synthetische Nukleinsäuresequenz ist ein Nukleinsäuremolekül, das die Nukleotidsequenz von SEQ ID NO: 2 umfasst oder aus dieser besteht. Ebenfalls Bestandteil dieser Erfindung sind Fragmente dieser Nukleotidsequenz, die für einen Teil des RSV F Proteins kodieren.

Im Vergleich zu der Wildtypsequenz mit SEQ ID NO: 1 konnten die Erfinder bei der Verwendung der Nukleotidsequenz von SEQ ID NO: 2 in menschlichen Zellen eine um den Faktor 1000 verbesserte Expression des F Proteins von RSV beobachten.

Die codon-optimierten Sequenzen werden dann in eine Expressionskassette eingebaut, die zusätzlich Sequenzen umfasst, die die effiziente Expression des kodierten Proteins in einer Wirtszelle, vorzugsweise einer menschlichen Zelle, erlauben. Eine solche Expressionskassette enthält dann die codon-optimierte Nukleotidsequenz mit damit funktional verknüpften zugehörigen Transkriptions- und Translationskontrollsequenzen, wie beispielsweise einem Promotor und/oder Terminierungssequenzen.

Die Bezeichnung "Kassette" oder "Expressionskassette" bezieht sich auf die erfindungsgemäßen Nukleotidsequenzen, die die Nukleotidsequenzen enthalten, die exprimiert werden sollen. Wegen entsprechender Restriktionsstellen an den 5'- und 3'-Enden der Sequenzen kann diese Kassette/Expressionskassette einfach in einen Vektor oder ein Plasmid eingeschoben, entfernt oder ersetzt werden. Die Expressionskassette enthält in der Regel zusätzlich zu der kodierenden Nukleotidsequenz Transkriptions- und Translationskontrollsequenzen, wie beispielsweise einen Promotor und/oder Terminierungssequenzen, die mit der Nukleotidsequenz funktional verbunden sind und die Expression in einer Wirtszelle erlauben.

Die Bezeichnung "Promotor" in Sinne der vorliegenden Erfindung bezieht sich auf eine Region auf einem DNA-Strang, der die Erkennung durch die RNA Polymerase ermöglicht. Nach der Erkennung der Promotorregion durch eine RNA-Polymerase wird durch die Bindung der RNA-Polymerase ein Initiationskomplex, um die Transkription einzuleiten. Dieser Komplex kann durch zusätzliche aktivierende Sequenzen (Enhancer) oder hemmende Sequenzen (Silencer) zusätzlich moduliert und reguliert werden.

Ein für die Expression der erfindungsgemäßen Nukleotidsequenzen geeigneter Promotor ist beispielsweise der CMV (Cytomegalievirus) Promotor. Dieser kann in einer Expressionskassette für die Expression in menschlichen Zellen funktional mit der erfindungsgemäßen Nukleotidsequenz verknüpft sein.

Eine solche Expressionskassette, die die erfindungsgemäße Nukleotidsequenz in Verbindung mit zusätzlichen für die Expression in einer Wirtszelle erforderlichen Sequenzen enthält, kann in einen Vektor eingefügt werden.

Die Bezeichnung "Vektor" bezieht sich auf Mittel, mit denen DNA-Moleküle in einen Wirtsorganismus oder ein Wirtsgewebe oder eine Wirtszelle eingebracht werden können. Es gibt verschiedene Arten von Vektoren, die beispielsweise Plasmide, Cosmide, Viren, z.B. Adenoviren, und Bakteriophagen einschließen.

Ein so erhaltener Vektor ist ebenfalls ein Bestandteil der vorliegenden Erfindung. Der Vektor ist vorzugsweise ein Plasmid oder ein viraler Vektor, insbesondere ein adenoviraler Vektor.

Ein solcher adenoviraler Vektor, der die erfindungsgemäße Nukleotidsequenz enthält, kann eine nicht-funktionelle E1-Genregion besitzen, um die Replikation des Virus und die Erzeugung infektiöser Viruspartikel in einer Wirtszelle zu verhindern. Vorzugsweise ist die E1-Genregion deletiert und/oder durch die eingefügte Expressionskassette ersetzt. Zusätzlich kann ein solcher Vektor auch eine Deletion der E3-Genregion aufweisen, die normalerweise für das Umgehen der Wirtsimmunität verantwortlich ist. Solche rekombinanten Adenoviren sind z. B. aus US 5,922,576 bekannt und können in bekannten Zelllinien ("packaging line"), wie z.B. 293-, 911- oder PerC.6-Zellen, die das virale E1-Gen exprimieren, vervielfältigt werden.

Um einen solchen adenoviralen Vektor herzustellen, kann die Expressionskassette, die die erfindungsgemäße Nukleotidsequenz umfasst, in einen Plasmidvektor eingefügt werden, z.B. über geeignete Restriktionsschnittstellen. Ein solcher Plasmidvektor kann adenovirale Nukleinsäuresequenzen umfassen, die die homologe Rekombination mit einem geeigneten Rückgratplasmid, das einen Großteil des adenoviralen Genoms enthält, ermöglichen. Durch die Rekombination erhält man dann einen adenovirales Transfer-(Shuttle)-Plasmid, das die erfindungsgemäße Nukleotidsequenz in Form einer Expressionskassette enthält. Ein solches adenovirales Transfer-(Shuttle-)Plasmid kann beispielsweise auch eine regulierte Expression der eingesetzten erfindungsgemäßen Nukleotidsequenz vermitteln.

Dadurch kann dann eine große Menge an adenoviralen Vektoren hergestellt werden. Die adenoviralen Vektoren können im Folgenden dann als Vakzin-Vektor verwendet werden.

Zur Herstellung von adenoviralen Vektoren, die das F-Protein exprimieren, kann der CMV-Promotor durch einen regulierbaren Promotor ersetzt werden. Damit kann der Vektor in Zellen expandiert werden, die ein Regulationssystem für die Expression des RSV F-Proteins, beispielsweise das Tetrazyklin regulierte Expressionsystem (293TRex Zellen), enthalten. Für die Herstellung und Expansion des adenoviralen Vektors werden daher beispielsweise 293TRex Zellen verwendet.

Regulierbare Promotoren sind Promotoren, die durch Substanzen, z.B. Antibiotika oder eines Proteins, das eine bestimmte DNA-Sequenz bindet, eine veränderte Expression des nachgeschalteten Transgen vermitteln. Solche regulierbaren Promotoren sind beispielsweise ein CMV-Promotor mit Bindestellen für den Tetrazyklin-Repressor (Tetrazyklin-regulierbarer Promotor) oder über das Steroid Ecdyson vermittelte regulierbare Expression, aber auch andere regulierbare Expressionssysteme.

Daher umfasst die vorliegende Erfindung in einem Aspekt auch Plasmide, beispielsweise oben beschriebene Shuttle-Plasmide, und adenovirale Vektoren, die die erfindungsgemäßen Nukleotidsequenzen enthalten. Der adenovirale Vektor enthält Fragmente der Nukleotidsequenz SEQ ID NO: 2 in Form einer Expressionskassette mit einem Promotor, beispielsweise dem CMV Promotor. Bevorzugt ist als ein solcher erfindungsgemäßer Vektor der adenovirale Vektor AdV-F_{syn}.

Die molekularbiologischen Standardtechniken zur Herstellung und Reinigung der DNA-Konstrukte der Erfindung, zur Herstellung der Adenoviren und adenoviralen Vektoren und der (Shuttle-) Plasmide sind dem Fachmann aus dem Stand der Technik bekannt.

Die adenoviralen Vektoren oder Plasmide, die die erfindungsgemäße Nukleotidsequenz enthalten, können einem Subjekt, beispielsweise einem Menschen, verabreicht werden, um eine Immunantwort gegen RSV zu induzieren.

Aus diesem Grund bezieht sich die vorliegenden Erfindung in einem weiteren Aspekt auf immunogene Zusammensetzungen, die die erfindungsgemäßen Nukleotidsequenzen mit Fragmenten von SEQ ID NO: 2 umfassen. In einer solchen Zusammensetzung kann die erfindungsgemäße Nukleotidsequenz in Form der oben erwähnten Plasmide oder Vektoren, vorzugsweise in Form eines adenoviralen Vektors, vorliegen. Ein solcher Vektor erlaubt den Transport der erfindungsgemäßen Nukleinsäuremoleküle in einen menschlichen Wirt und die Expression des kodierten Proteins. Das Protein wird dabei in einer Menge exprimiert, die ausreicht die beabsichtigte Immunantwort auszulösen.

Die immunogenen Zusammensetzungen können abhängig von der gewünschten Art der Anwendung auch einen pharmazeutisch annehmbaren Träger- und/oder Hilfsstoff enthalten. Hilfsstoffe umfassen unter anderem auch bekannte Adjuvantien.

Die Verabreichung der immunogenen Zusammensetzungen kann dabei auf dem Fachmann bekanntem Weg erfolgen und schließt orale Dosierungsformen, wie z.B. Tabletten, Kapseln, Pulver, Granulate, Lösungen, Suspensionen, Sirups und Emulsionen, oder alternativ Injektionen, beispielsweise intravenös, intraperitoneal, subkutan oder intramuskulär, ein. Ebenfalls möglich sind die intranasale oder inhalative Verabreichung. Alle diese Verabreichungswege sind dem Fachmann auf dem Gebiet bekannt. Bevorzugte Verabreichungswege sind oral, intranasal, inhalativ, subkutane oder intramuskuläre Injektion

Die Formulierung der immunogenen Zusammensetzungen in einer Form, die für die gewünschte Art der Verabreichung geeignet ist, ist dem Fachmann bekannt und kann beispielsweise Remington: The Science and Practice of Pharmacy ("Remington's Pharmaceutical Sciences") von Gennaro A.R., 20te Ausgabe, 2000: Williams & Wilkins PA, USA, entnommen werden. Beispielsweise können die erfindungsgemäßen Nukleinsäuremoleküle in einer physiologisch akzeptablen Lösung, wie z.B. steriler Kochsalzlösung oder steril gepufferter Salzlösung vorliegen.

Eingeschlossen ist daher auch die Verwendung der erfindungsgemäßen Nukleinsäuremoleküle, Plasmide oder Vektoren für die Herstellung einer Impfstoffzusammensetzung für die Impfung eines Subjekts gegen durch die Infektion mit RSV hervorgerufene Erkrankungen. Dieses Subjekt ist vorzugsweise ein Mensch. Die Impfung kann beispielsweise in einem "prime and boost"-Verfahren erfolgen.

Die Menge an exprimierbarer DNA, die bei einer Impfung in den Empfänger eingebracht werden soll, hängt teilweise von der Stärke des verwendeten Promotors und der Immunogenität des exprimierten Genprodukts ab. Im Allgemeinen wird für eine immunologisch oder prophylaktisch effektive Dosis eines Plasmid-Vakzin-Vektors eine Dosis von 1 ng bis 100 mg, vorzugsweise von ungefähr 10 µg bis 300 µg, direkt in das Muskelgewebe verabreicht. Eine effektive Dosis für rekombinante Adenoviren sind in etwa 10⁶-10¹² Partikel, bevorzugt 10⁷-10¹¹ Partikel.

Ein Verfahren zur Herstellung des RSV F-Proteins, wobei das Verfahren die Expression eines erfindungsgemäßen Nukleinsäuremoleküls in einer geeigneten Wirtszelle umfasst ist ebenfalls Bestandteil der vorliegenden Erfindung. Das erfindungsgemäße Nukleinsäuremolekül ist für die Expression in der Wirtszelle codon-optimiert. Dieses Verfahren umfasst die Transfektion einer erfindungsgemäßen Nukleinsäuresequenz, beispielsweise in Form eines Plasmids, in eine geeignete Wirtszelle, Expression des RSV-F Proteins in der Wirtszelle und, gegebenenfalls, die Isolierung und Reinigung des RSV-F Proteins aus den Zellen. Das Verfahren ist vorzugsweise ein *in vitro* Verfahren.

Weiter hierin beschrieben sind die durch die Expression der Nukleinsäuremoleküle mit SEQ ID NO: 1 oder 2 oder Fragmente davon erhaltenen Polypeptidmoleküle. Diese Polypeptide umfassen oder bestehen aus der Aminosäuresequenz mit SEQ ID NO: 3 oder Fragmenten davon. Diese Polypeptidmoleküle können in immunogenen Zusammensetzungen enthalten sein und zur Impfung verwendet werden.

"Fragment" im Zusammenhang mit einem Polypeptid bezieht sich auf C- und/oder N-terminal verkürzte Proteine. Die daraus resultierenden Peptide sind vorzugsweise immunogen und mindestens 10 oder mehr, vorzugsweise 20 oder mehr, noch bevorzugter 30 oder mehr Aminosäuren lang.

Die nachfolgenden Beispiele dienen der genaueren Veranschaulichung der Erfindung und sind nicht dazu bestimmt, die Erfindung in irgendeiner Weise zu beschränken.

### Beispiele

### Beispiel 1: Kultivierung von RSV

Die Passage von RSV wurde mit Hep2 Zellen durchgeführt und bei -80°C gelagert. Hep2 oder 293T Zellen wurden durch die Zugabe von RSV-enthaltendem Zellüberstand infiziert. Zwei Stunden nach der Zugabe des Virus wurden die Überstände entfernt und die Zellen mit DMEM Medium, das 0,5% fötales Kälberserum (FCS) und 100 µg/ml Penicillin G und Streptomycinsulfat enthielt, versorgt.

### Beispiel 2: Herstellung des RSV-F Expressionsplasmids

Für die Herstellung des RSV-F Expressionsplasmids, wurde unter Verwendung des Qiagen RNeasy® Mini Kits aus RSV-infizierten Hep2 Zellen cytoplasmatische RNA isoliert. Nach der reversen Transkription (ThermoScript^{™} RT-PCR System, Invitrogen) wurde die RSV-F cDNA über PCR (Primers: sense: 5'- [G A T C C A A G C T T C C A C C] A T G G A G T T GCCAATCCTCAAA; antisense: 5'-[TCGACCTCGAG]TTAGTTACTAAATGCAA T A T T A T T T A T A C C) unter Verwendung der Platinum® Taq DNA-polymerase (Invitrogen) amplifiziert. Das 1.7 kb Fragment wurde in den pcDNA3.1(+) Vektor (Invitrogen) umkloniert.

Die Codon-Optimierung des offenen Leserahmens (ORF) des Wildtyps wurde von der Firma Geneart (Regensburg, Deutschland) durchgeführt. Dabei wurden synthetisch hergestellte Oligonukleotide zusammengefügt und das resultierende Fragment in das pUC57 Plasmid einkloniert und sequenziert.

Der codon-optimierte ORF wurde in den pcDNA3.1(+) Vektor (Invitrogen) und den pI Vektor über HindIII / Xhol Restriktion umkloniert. Die Deletion des Stop Codons des RSV-F ORF wurde mit PCR Mutagenese durchgeführt. Die Sequenz aller Plasmide wurde über eine Sequenzanalyse (Genterprise, Mainz, Deutschland) bestätigt.

### Beispiel 3: Zelltransfektion

293T und Hep2 Zellen wurden in Dulbecco's modified Eagle's medium (Invitrogen) ergänzt mit 10% fötalem Kälberserum (Invitrogen), Penicillin G and Streptomycinsulfat in einer Endkonzentration von jeweils 100 µg/ml kultiviert. Die Zellen wurden in 25 cm² Kolben mit 5 µg der in Beispiel 2 hergestellten Plasmid-DNA mittels der Calciumphoshat Kopräzipitationsmethode, wie von DuBridge et al. (DuBridge et al. (1987) Analysis of mutation in human cells by using an Epstein-Barr virus shuttle system. Mol. Cell Biol., 7, 379-387) beschrieben, transfiziert.

### Beispiel 4: Homologe Rekombination mittels Elektroporation und Herstellung elektrokompetenter BJ5183-Bakterien

Zur Herstellung von rekombinanten adenoviralen Vektoren wurde die Methode der homologen Rekombination eingesetzt. Es wurden ein linearisierter Shuttle-Vektor (Figur 13) und ein adenoviraler Vektor (pAdEasy-1; Q BIOgene, Carlsbad, CA, USA) mittels Elektroporation kotransformiert. Aufgrund homologer Arme in den Plasmiden erfolgte eine Rekombination in BJ5183 Bakterien; das entstehende Plasmid trug eine Kanamycin-Resistenz, wodurch positive Klone auf einer LB_{Kan}-Agarplatte selektioniert werden konnten. Ein solches Vektorsystem ist beispielsweise auch unter dem Namen AdEasy^{™} von Q BIOgene (Carlsbad, CA, USA) erhältlich.

Zur Herstellung elektrokompetenter BJ5183-Bakterien wurde eine frische Kolonie oder ein gefrorener Stock der Bakterien wurde über Nacht in 10 ml LB-Medium mit Streptomycin angezogen; diese Vorkultur wurde verwendet, um eine 1 1-Kultur anzuimpfen. Die Bakterien wurden bei 37°C im Horizontalschüttler bei 200 - 220 rpm bis zu einer OD₆₀₀ von 0,6 - 0,8 (ca. 3 Stunden) geschüttelt. Danach wurden die Zellen in Zentrifugationsbecher gesammelt und 60 min auf Eis inkubiert. Nach der Inkubation wurden die Zellen bei 2.600 g und 4°C für 10 min pelletiert, dann mit 11 eiskaltem Wasser mit 10% Glycerol gewaschen und für 30 min pelletiert. Nach Wiederholen des Waschschrittes wurden die Zellen in 20 ml Restvolumen resuspendiert, in ein 50 ml-Gefäß überführt und erneut für 10 min pelletiert. Nun wurden die Zellen in 3 - 5 ml Restvolumen resuspendiert, aliquotiert, in flüssigem Stickstoff eingefroren und bei -80°C gelagert.

Für die homologe Rekombination wurden auf Eis in einer gekühlten Elektroporations-Küvette 40 µl BJ5183-Bakterien vorgelegt und etwa 100 ng Plasmid-DNA des adenoviralen Vektors und etwa 1 µg Plasmid-DNA des Shuttle-Vektors dazu pipettiert. Die Elektroporation erfolgte bei einer Spannung von 2500 V. Nach Zusatz von 300 µl SOC-Medium wurden die Bakterien bei 37°C für 1 h in einem Schüttelapparat inkubiert und anschließend auf LB_{Kan}-Platten über Nacht ausplattiert.

Von den gewachsenen Klonen wurde die DNA in analytischem Maßstab isoliert und überprüft. Bei positiver Rekombination wurde sie in DH5α-Bakterien retransformiert, um weitere Rekombinationsereignisse zu verhindern. Dazu wurden 50 µl DH5α Bakterien mit 1 µl DNA versetzt und elektroporiert.

### Beispiel 5: Präparative Gewinnung der in vitro klonierten oder in BJ5183-Bakterien rekombinierten Plasmid-DNA

Zur präparativen Gewinnung der *in vitro* klonierten oder in BJ5183-Bakterien rekombinierten Plasmid-DNA dienten die Escherichia coli Stämme XL2-Blue (Stratagene) und DH5α (Invitrogen). Die verwendeten Plasmide ermöglichten mittels eines Resistenzgens die Selektion durch entsprechende Antibiotika (Ampicillin oder Kanamycin). Die transformierten Bakterien wurden auf LB-Agarplatten und in LB-Flüssigmedium mit einem Zusatz des jeweiligen Antibiotikums selektiert. Als Vorkultur für präparative Plasmidisolierungen dienten 3 ml Übernachtkulturen, die bei 37°C in einem Horizontalschüttler inkubiert wurden. Zur Extraktion größerer Mengen an DNA wurden 100 ml oder 250 ml LB-Medium in sterilen Erlenmeyerkolben mit 1 ml einer Vorkultur angeimpft und bei 37°C über Nacht im Horizontalschüttler bei 200 - 220 rpm inkubiert.

### Beispiel 6: Calciumphosphat-Transfektion von Zellen

Zur Anzucht adenoviraler Vektoren mussten die hergestellten adenoviralen Plasmide (nach Linearisierung durch PacI - Verdau und anschließendem Aufreinigen durch Phenol / Chloroform-Extraktion) in T-REx^{™}-293-Zellen (Invitrogen) transfiziert werden. Dazu wurde die Methode der Calciumphosphat-Kopräzipitation verwendet, wobei die DNA über Salzkomplexe an die Zellmembran bindet und aufgenommen wird.

1 Tag vor der Transfektion wurden 800000 T-REx-Zellen aus einer logarithmisch wachsenden Kultur entnommen, mit 5 ml Medium in eine frische 25 cm²-Zellkulturflasche ausgesät und über Nacht bei 37°C im Brutschrank inkubiert.

Die Zellen waren zum Zeitpunkt der Transfektion circa 50 % konfluent. 1-4 Stunden vor der Transfektion wurde das Zellkulturmedium durch frisches Medium ersetzt. Für die Transfektion wurden 15-20 µg DNA mit 169 µl sterilem H₂O auf Eis vorgelegt. Dazu wurden 31 µl 2 M CaCl₂ pipettiert und sorgfältig vermischt. Bei Raumtemperatur wurden 250 µl 2x HBS-Puffer-Lösung tropfenweise zur H₂O-DNA-CaCl₂-Mischung gegeben. Die anschließende zehnminütige Inkubation bei Raumtemperatur ermöglichte die Ausbildung eines milchigen Präzipitats. Danach wurde der Transfektionsansatz noch einmal durchmischt und in das Zellkulturmedium getropft.

Nach maximal 18 Stunden Inkubation im Brutschrank wurde das Medium gewechselt, da das CaCl₂ toxisch auf die Zellen wirken kann.

In eine zweite Zellkulturflasche wurde jeweils parallel das Plasmid für AdV-eGFP (adenoviraler Vektor, der das eGFP-Protein exprimiert) transfiziert, sodass im Fluoreszenzmikroskop anhand der sich ausbildenden Fluoreszenz infizierter Zellen der Erfolg der Transfektion und der Fortschritt der Produktion von Adenoviren verfolgt werden konnte. Eine Woche später waren in der Regel alle Zellen durchlysiert, der Überstand mit den produzierten Adenoviren wurde abgenommen und konnte für die nächste Infektion zur weiteren Anzucht der adenoviralen Vektoren verwendet werden.

### Beispiel 7: Anzucht von Adenoviren

Die adenoviralen Vektoren wurden in T-REx^{™}-293-Zellen (Invitrogen) angezüchtet. Diese Zelllinie wurde gewählt, da sie wegen ihres Tetrazyklin-regulierten Expressionssystems zur Anzucht von Adenoviren, die ein zelltoxisches Gen, wie das Fusionsprotein von RSV exprimieren, geeignet ist. So kommt es in anderen Zelllinien bei der Adenovirusvermehrung durch die Expression des RSV-F-Proteins zur Syncytienbildung in der Zellkultur, wodurch die Virusproduktion vermindert wird. In den T-REx-Zellen dagegen wird bei der Virusvermehrung die Expression des RSV-F-Proteins in Abwesenheit von Tetrazyklin unterdrückt, die Virusausbeute ist deutlich größer.

Die Anzucht von Adenoviren in T-REx-Zellen verlief über mehrere Infektionszyklen. Bei den ersten Passagen wurde jeweils eine 75 cm² große Zellkulturflasche infiziert, später jeweils eine 175 cm² große Zellkulturflasche. Zur Infektion wurden jeweils 3-8 ml des eine Passage zuvor geernteten Zellkulturüberstands in einer geeigneten Menge frischen Mediums verwendet. Zum Zeitpunkt der Infektion waren die Zellkulturen dabei circa 70 %ig konfluent. In den ersten vier Stunden nach Viruszugabe wurden die Zellen regelmäßig geschwenkt, um die Infektionsraten zu erhöhen. Dann erfolgte ein Mediumwechsel (10-15 ml Medium bei der 75 cm² großen Flasche, 20 ml bei der 175 cm² großen Flasche).

Der Fortschritt der Infektion wurde täglich kontrolliert und zeigte sich am CPE (cytopathischer Effekt), welcher als Abkugeln der Zellen ersichtlich ist. Die Virusernte erfolgte nach 3-6 Tagen, sobald alle Zellen durchinfiziert und abgeschwommen waren. Dabei wurde der Zellkulturüberstand abgenommen und anschließend dreimal abwechselnd bei -80°C eingefroren und im 25°C-Wasserbad aufgetaut, damit auch die Zellen, die bisher noch nicht aufgeplatzt waren, die produzierten Adenoviren freisetzten. Nach den drei Einfrier/Auftau-Zyklen wurde der Zellschrott bei 1000g und 4°C für 10 Minuten abzentrifugiert. Der Überstand mit den Adenoviren wurde bei -80°C gelagert und für die nächste Infektion verwendet.

Die adenoviralen Vektoren wurden solange in T-REx-Zellen passagiert, bis ein möglichst hoher Titer (bestimmt nach dem Verfahren der GTU) von idealerweise mindestens 10¹⁰ GTU / ml erreicht wurde. Dann wurden erst 2-3 175 cm² große Zellkulturflaschen infiziert und mit dem davon geernteten Überstand 5 oder 10 175 cm² große Zellkulturflaschen, um genügend Ausgangsmaterial für die anschließende Virusaufreinigung zu erhalten.

### Beispiel 8: Aufreinigung und Konzentrierung von Adenoviren

Die Aufreinigung und Konzentrierung der adenoviralen Vektoren erfolgte mit dem Kit Vivapure® AdenoPACK™ 100 (Sartorius) entsprechend der Anleitung des Herstellers. Dabei würde eine Konzentrierung um den Faktor 1000 erreicht. Der Pufferaustausch-Schritt im Rahmen der Schlusskonzentrierung wurde durchgeführt, wobei die gewonnenen Adenoviren in Storage Buffer aufgenommen wurden.

### Beispiel 9: Titerbestimmung adenoviraler Präparationen

Zur Titerbestimmung adenoviraler Vektoren wurden zwei verschiedene Methoden verwendet.

Bei der Bestimmung der OPU (optische Partikel-Einheiten) wird der Titer der adenoviralen Präparation über eine photometrische DNA-Bestimmung ermittelt. Dabei werden alle DNA-enthaltenden viralen Partikel erfasst, das heißt auch defekte Partikel, die nicht infektiös sind oder die nicht das gewünschte Gen tragen. Vorteile der OPU-Bestimmung sind, dass diese Methode der Titerbestimmung die schnellste ist und die höchste Reproduzierbarkeit aufweist.

Zur Bestimmung der OPU wurde eine Verdünnungsreihe mit beispielsweise den Verdünnungen 1:2, 1:5, 1:10, 1:20, 1:25 und 1:50 von der adenoviralen Präparation hergestellt. Dabei wurden die aufgereinigten Adenoviren jeweils in Virion Lysis Buffer (0,1 % SDS, 10 mM Tris-HCl pH 7,4, 1 mM EDTA in H₂O) verdünnt. Für jede Verdünnungsstufe wurde eine Blanklösung hergestellt, indem analog Storage Buffer (10 % Glycerin in Dulbecco's modified PBS) (anstatt der adenoviralen Präparation) in Virion Lysis Buffer verdünnt wurde. Nach einer Inaktivierung der Virus-haltigen Proben bei 56°C für 10 Minuten wurde die Absorption der Verdünnungen bei der Wellenlänge λ=260 nm (OD₂₆₀) gemessen, wobei das Photometer zuvor jeweils mit der zugehörigen Blanklösung eingestellt wurde. Die OPU wurde anschließend aus den OD₂₆₀-Werten nach der folgenden Formel, bei der als Extinktionskoeffizient der Wert 1,1 x 10¹² [74] gewählt wird, bestimmt: OPU / ml = OD₂₆₀ x Verdünnung der Präparation x 1,1 x 10¹² Dabei wurde die OPU als Durchschnitt der für die einzelnen Verdünnungen berechneten Werte ermittelt.

Da die OPU (wie oben beschrieben) auch defekte Partikel einschließt, wurde zusätzlich die GTU (gentrarisduzierende Einheiten), das heißt der Anteil der infektiösen und gentransduzierenden und somit gewünschten adenoviralen Vektoren an der OPU, anhand einer seriellen Verdünnungsreihe auf 293A-Zellen mit anschließender spezifischer immunzytochemischer Färbung des auf infizierten Zellen exprimierten RSV-F-Proteins bestimmt.

Die Titration wurde auf 293A-Zellen durchgeführt. Dazu wurde eine 96-Well-Platte Typ F mit circa 10000 293A-Zellen (in 100 µl Medium) pro Well belegt und im Brutschrank kultiviert. Einen Tag später wurde die Titerplatte infiziert. Dabei wurde die adenovirale Präparation im Verhältnis 1:1000 ins erste Well der Titerplatte gegeben (10 µl einer 1:100 Vorverdünnung wurden zu den 100 µl Medium im ersten Well hinzugegeben) und horizontal sukzessive von Well zu Well seriell 1:10 weiter verdünnt (jeweils 10 µl wurden von Well zu Well transferiert). Diese Verdünnungsreihe wurde wiederholt in 3-5 Reihen durchgeführt. 2-3 Reihen blieben als Negativkontrolle Virus-frei und wurden analog verdünnt.

2-3 Tage nach der Infektion der Titerplatte wurde eine spezifische immuncytochemische Färbung des F-Proteins von RSV durchgeführt. Bei Testung des adenoviralen Vektors AdV-F_{syn} (adenoviraler Vektor, der die Nukleotidsequenz von SEQ ID NO: 2 enthält) war dies direkt möglich, da die infizierten Zellen das RSV-F-Protein auf ihrer Oberfläche exprimieren.

Bei der Färbung der Titerplatte wurde im ersten Schritt das Medium abgenommen und die Zellen für 10 Minuten mit 100 µl 80 %igem Ethanol pro Well fixiert. Nach Abnehmen des Ethanols wurde die Platte an der Luft getrocknet. Dann wurden zur Rehydrierung 200 µl PBS-T_{0,05 %} (0,05 % (v/v) Tween 20 in PBS) in jedes Well gegeben und 5 Minuten lang auf der Platte belassen. Die Platte wurde ausgeklopft, dann wurden in jedes Well 100 µl einer 1:250 Verdünnung des 18F12-Erstantikörpers in PBS-T_{0,05} % gegeben. Die Platte wurde anschließend für 1 Stunde bei 37°C inkubiert. Dabei bindet der 18F12-Antikörper, der gegen das F-Protein von RSV gerichtet ist, an das auf infizierten Zellen exprimierte F-Protein. In einem dreimaligen Waschschritt mit PBS-T_{0,05 %} wurde nicht gebundener Antikörper entfernt. Zur Detektion des Erstantikörpers wurde als Enzym-gekoppelter Zweitantikörper Kaninchen anti-Maus Immunglobulin P0260 (Dako), welches an den monoklonalen 18F12-Antikörper bindet, verwendet und 1:400 in PBS-T_{0,05 %} verdünnt. Von dieser Lösung wurden 100 µl in jedes Well gegeben, die Platte wurde erneut für 1 Stunde bei 37°C inkubiert. Nach einem dreimaligen Waschschritt mit PBS-T_{0,05 %} wurde die durch das am Sekundärantikörper gebundene Enzym vermittelte Farbreaktion durch Zugabe von 100 µl der Färbe-Lösung (200 µl AEC (3-Amino-9-ethylcarbazol 10mg/ml gelöst in 96% Ethanol) und 10 µl H₂O₂ in 10 ml Phosphat-Citrat) in jedes Well gestartet. Die Farbreaktion erfolgte bei einer Inkubation bei 37°C für 30 Minuten und wurde durch Zugabe von 100 µl H₂O pro Well nach Ausklopfen der Platte gestoppt.

Die Interpretation erfolgte unter dem Mikroskop, indem für jede Reihe die Anzahl der gefärbten Zellen in dem letzten Well, in dem anhand der Anfärbung noch eine Infektion nachgewiesen werden konnte, bestimmt und mit der in diesem Well vorliegenden Verdünnungsstufe multipliziert wurde. Die GTU berechnet sich aus dem Durchschnitt aller Reihen.

Die oben beschriebene Färbemethode bezieht sich auf die Titerbestimmung von Präparationen RSV-F-Protein exprimierender adenoviraler Vektoren. Bei der Bestimmung des Titers einer Präparation von AdV-eGFP dagegen wurde keine Färbung durchgeführt, die Titerplatte wurde bei Betrachtung im Fluoreszenzmikroskop durch Zählung der grün leuchtenden Zellen ausgewertet.

### Beispiel 10: Immunisierungsstudie im BALB/c-Mausmodell

Im Rahmen einer ersten Immunisierungsstudie wurde eine Gruppe von 6 BALB/c-Mäusen subkutan mit 1 x 10⁸ GTU (5 x 10⁹ OPU) AdV-F_{syn} immunisiert. Ein Boost erfolgte nach 4 Wochen. Zum Vergleich wurde eine Gruppe von ebenfalls 6 Mäusen subkutan mit Plasmid-DNA immunisiert. Dabei wurde das Plasmid pcDF_{syn} ED verwendet, welches für die Ektodomäne des synthetischen F-Proteins von RSV kodiert und in Vorversuchen mit BALB/c-Mäusen im Vergleich mit den Plasmiden pcIF_{syn} und pcIF_{wt}, die für das synthetische F-Protein (Volllänge) beziehungsweise das Wildtyp-F-Protein von RSV kodieren, den besten Schutz nach DNA-Immunisierung vor RSV induziert hatte (Figur 1). FI-RSV diente hier als Kontrolle der Induktion der Immunantwort gegen RSV und bezeichnet die zur Immunisierung verwendeten mit Formalin inaktivierten RS-Viren. Als Kontrollgruppen wurden 6 Mäuse subkutan mit AdV-OVA (adenoviraler Vektor, der Ovalbumin exprimiert) immunisiert, 3 Mäuse wurden überhaupt nicht immunisiert. Drei Wochen nach der zweiten Immunisierung wurden die Mäuse intranasal mit ca. 1 x 10⁷ iE (infektiöse Einheiten) von Plaque aufgereinigtem RSV infiziert.

Am Tag 5 nach Infektion wurden die Mäuse nach Betäubung getötet und eine BAL (bronchoalveoläre Lavage) gewonnen. Aus der BAL wurde die virale RNA extrahiert und als Maß für den Schutz der Tiere der RSV-RNA-Gehalt mittels quantitativer RT-PCR bestimmt. Bei den Kontrollgruppen wurde mit durchschnittlich circa 20000 RSV-Kopien eine hohe RSV-Beladung bestimmt. Während nach Immunisierung mit pcDF_{syn} ED die RSV-Kopienzahl bereits deutlich um einen Faktor von circa 55 (durchschnittlich 360 Kopien pro BAL) reduziert war, war nach Immunisierung mit AdV-F_{syn} bei allen Mäusen der Gruppe überhaupt kein RSV mehr nachweisbar, das heißt, die RSV-Beladung wurde hier um einen Faktor von mindestens etwa 400 reduziert, da die Nachweisgrenze bei der qRT-PCR bei 50 Kopien liegt. Somit sind die Tiere nach Impfung mit pcDF_{syn} ED nur bedingt vor RSV geschützt, da die RSV-Replikation in den Lungen der Mäuse nur gehemmt, nicht aber vollständig verhindert wird. Nach Impfung mit AdV-F_{syn} dagegen scheinen die Tiere vollständig geschützt zu sein, da kein RSV mehr nachweisbar ist (Figur 2).

Im Verlauf des Versuchs wurde den Mäusen in der Woche 0 (vor der ersten Immunisierung) und 7 (nach der zweiten Immunisierung) sowie am Tötungstag Blut entnommen und davon Serum gewonnen. Eine zusätzliche Blutentnahme nach der ersten und vor der zweiten Immunisierung wurde in dieser ersten Immunisierungsstudie noch nicht durchgeführt.

Mittels der Serumproben wurde im Antikörper-ELISA der Titer an RSV-spezifischen IgG1- und IgG2a-Antikörpern im Blut der Mäuse bestimmt. Da eine Standardisierung dieses ELISAs bisher noch nicht möglich war, können die Ergebnisse hierbei nur innerhalb der Gruppen einer Immunisierungsstudie verglichen werden. Dabei werden alle Proben einer Immunisierungsstudie zeitgleich auf einer Elisa-Platte untersucht.

Vor Immunisierung konnten bei keiner Mäusegruppe RSV-spezifische IgG1- oder IgG2a-Antikörper nachgewiesen werden. In den Kontrollgruppen blieb der Titer im Verlauf des Experiments gleich niedrig. Bei den mit AdV-F_{syn} oder mit pcDF_{synED} geimpften Mäusen dagegen konnte nach der zweiten Immunisierung und am Tötungstag ein klarer Anstieg der RSV-spezifischen Antikörper gezeigt werden. Während die IgG1-Antikörper bei beiden Gruppen auf ähnliche Werte anstiegen, war die Erhöhung der IgG2a-Antikörper nach Impfung mit AdV-F_{syn} vielfach stärker als nach Impfung mit pcDF_{synED}. Somit bewirkt die zweifache Immunisierung mit AdV-F_{syn} eine starke Immunantwort hinsichtlich der Bildung systemischer RSV-spezifischer IgG-Antikörper und induziert hierbei die Herstellung von weitaus mehr IgG2a als nach zweifacher Immunisierung mit pcDF_{synED} (Figur 3). Dass bei den mit AdV-F_{syn} geimpften Mäusen die IgG2a-Antikörper vielfach stärker als die IgG1-Antikörper ansteigen, deutet auf eine T_{H}1-basierte Immunantwort nach Immunisierung mit AdV-F_{syn} hin, was als besonders positiv beurteilt werden kann, da die in den 60er Jahren nach Immunisierungsversuchen mit Formalin-inaktiviertem RSV beobachtete Krankheitsverschlimmerung vor allem auf eine Verschiebung der Immunantwort zugunsten einer T_{H}2-Antwort zurückgeführt wurde.

Die Seren der Mäuse wurden außerdem im Neutralisationstest auf neutralisierende Antikörper gegen RSV getestet. Dabei waren zu Beginn des Versuchs in keiner Mäusegruppe neutralisierende Antikörper vorhanden. Während in den Kontrollgruppen bei allen Serumproben keine neutralisierenden Antikörper gegen RSV gefunden werden konnten, führte die Immunisierung mit AdV-F_{syn} oder pcDF_{synED} zur Bildung neutralisierender Antikörper. Dabei stiegen diese nach AdV-F_{syn}-Impfung ungefähr 13fach stärker an als nach Impfung mit pcDF_{synED}, sodass die Immunisierung mit AdV-F_{syn} auch hier als der Impfung mit pcDF_{synED} überlegen angesehen werden kann. Im Vergleich zu den Serumproben nach der zweiten Immunisierung fand sich bei den Serumproben vom Tötungstag, das heißt nach Challenge, jeweils eine leichte Verminderung des Titers neutralisierender Antikörper, was durch einen Verbrauch dieser im Verlauf der Infektion erklärt werden kann (Figur 4).

In den durchgeführten Immunisierungsstudien konnte demnach die Überlegenheit von AdV-F_{syn} über pcDF_{synED} deutlich gezeigt werden (Induktion eines höheren Titers an RSV-spezifischen Antikörpern, geringere RSV-Beladung in der Lunge und entsprechend besserer Schutz der Mäuse).

### Beispiel 11: Immunisierung mit AdV-F_{syn} über verschiedene Administrationsrouten

In diesem Experiment sollte der Einfluss der Administrationsroute auf die Immunisierung mit AdV-F_{syn} untersucht werden. Dazu wurden drei Gruppen von je 6 BALB/c-Mäusen mit 1 x 10⁸ GTU (5 x 10⁹ OPU) AdV-F_{syn} immunisiert, wobei der Impfstoff bei der ersten Gruppe intranasal, bei der zweiten intramuskulär und bei der dritten subkutan verabreicht wurde. Eine vierte Gruppe mit ebenfalls 6 BALB/c-Mäusen wurde als Negativkontrolle nicht immunisiert. Das Zeitschema für Impfungen, Blutentnahmen und Challenge wurde von der ersten Immunisierungsstudie übernommen (siehe Beispiel 10).

Am Tötungstag (5 Tage nach Infektion) wurde die BAL der Mäuse gewonnen. Nach Isolierung viraler RNA aus der BAL wurde wieder der RSV-RNA-Gehalt mittels quantitativer RT-PCR bestimmt, um eine Aussage über den Schutz der Tiere nach Impfung treffen zu können. Während die RSV-Beladung in der nicht-immunisierten Kontrollgruppe sehr hoch war, wiesen alle drei mit AdV-F_{syn} geimpften Mäusegruppen einen sehr guten Schutz auf. Abgesehen von einer einzigen Maus aus der intramuskulär geimpften Mäusegruppe, bei der die RSV-Beladung in der BAL bei 88 Kopien lag, war in keiner BAL eine RSV-Beladung nachweisbar, das heißt, dass alle Mäuse unabhängig von der Administrationsroute nach Impfung mit AdV-F_{syn} sehr gut geschützt waren (Reduktion um einen Faktor von mindestens 160 in allen drei Mäusegruppen) (Figur 5).

Da bei der quantitativen RT-PCR, die anhand der RNA-Isolate aus den BALs der Mäuse durchgeführt wurde, keine Unterschiede bezüglich der RSV-Beladung in den Lungen der verschiedenen Mäusegruppen festgestellt werden konnten, wurde zusätzlich die Gesamt-RNA aus dem Lungenhomogenat, welches ebenfalls am Tötungstag gewonnen worden war, isoliert. Diese RNA-Isolate wurden ebenfalls in der quantitativen RT-PCR auf ihren RSV-Gehalt getestet, um einen zweiten Wert für die RSV-Beladung in den Mäuselungen und somit für den Schutz der Mäuse vor RSV zu erhalten. Dabei wurden die Ergebnisse der qRT-PCR anschließend in Bezug auf den RNA-Gehalt des RNA-Isolats umgerechnet, da bei der RNA-Isolierung nicht nur virale RNA, sondern die Gesamt-RNA isoliert wurde.

Bei der quantitativen RT-PCR wurde so in der nicht-immunisierten Kontrollgruppe eine RSV-Kopienzahl von durchschnittlich circa 160000 Kopien / µg RNA bestimmt. Die Bestimmung der RSV-Beladung anhand von RNA-Isolaten aus dem Lungenhomogenat ist somit wesentlich sensitiver als die anhand von RNA-Isolaten aus der BAL, wo in der nicht-immunisierten Kontrollgruppe nur eine RSV-Beladung von durchschnittlich circa 8000 Kopien / BAL gefunden wurde. Dementsprechend zeigten sich hier auch Unterschiede in den immunisierten Mäusegruppen. Der beste Schutz wurde nach intranasaler Immunisierung mit AdV-F_{syn} erreicht. Hier lag die RSV-Beladung in der qRT-PCR bei allen Mäusen unterhalb der Nachweisgrenze von 50 Kopien. Umgerechnet in Bezug zum RNA-Gehalt der Proben heißt das, dass die intranasal geimpften Tiere nur eine sehr geringe RNA-Beladung aufwiesen. Sogar unter Annahme von 50 Kopien als Ergebnis der qRT-PCR war 1 Tier komplett geschützt, während die anderen 5 Tiere 1-5 Kopien / µg RNA aufwiesen. Somit wird durch die intranasale Immunisierung ein sehr guter, nahezu kompletter Schutz vor RSV induziert, wobei die RSV-Beladung um einen Faktor von circa 70000 reduziert wird. Nach intramuskulärer und subkutaner Impfung mit AdV-F_{syn} dagegen war eine RSV-Beladung in der qRT-PCR nachweisbar. Pro Gruppe waren nur jeweils 2 Tiere mit einer RSV-Beladung von 2 Kopien / µg RNA sehr gut geschützt, die jeweils anderen 4 Tiere zeigten eine durchschnittliche RSV-Beladung von circa 570 Kopien / µg RNA nach intramuskulärer Impfung (circa 280fache Reduktion) beziehungsweise circa 275 Kopien / µg RNA nach subkutaner Impfung (circa 580fache Reduktion) (Figur 6). Somit ist die intranasale Route bei einer Immunisierung mit AdV-F_{syn} eindeutig am besten geeignet, um einen sehr guten Schutz vor RSV zu induzieren.

Die im Verlauf des Experiments gewonnenen Seren der Mäuse wurden wieder im IgG-Antikörper-ELISA und im Neutralisationstest auf die Induktion systemischer RSV-spezifischer Antikörper getestet.

Vor der Immunisierung waren in keiner Mäusegruppe RSV-spezifische IgG1- oder IgG2a-Antikörper im ELISA nachweisbar. In der Kontrollgruppe blieb der Titer auch bei den weiteren Serumproben ähnlich niedrig. Bei den drei mit AdV-F_{syn} geimpften Mäusegruppen dagegen fand sich ein deutlicher RSV-spezifischer Titeranstieg sowohl bei IgG1- als auch bei IgG2a-Antikörpern. Dabei war dieser Anstieg von der Administrationsroute von AdV-F_{syn} abhängig, da der Antikörpertiter in den intranasal (i.n.), intramuskulär (i.m.) und subkutan (s.c.) geimpften Mäusen jeweils unterschiedlich ausfiel. So variierte der Titer vor allem der IgG2a-Antikörper recht stark, die subkutane Immunisierung führte zu einem vielfach stärkeren IgG2a-Antikörperanstieg als die intranasale oder die intramuskuläre Impfung. Die Unterschiede im IgG1-Titer waren dagegen nicht so stark ausgeprägt, da die Induktion von IgG1-Antikörpern bei allen Mausgruppen schwächer ausfiel als die der IgG2a-Antikörper. Trotzdem ist ersichtlich, dass hier die subkutane Immunisierung den schwächsten IgG1-Anstieg bewirkte. Bereits im Rahmen der ersten Immunisierungsstudie konnte gezeigt werden, dass die subkutane AdV-F_{syn}-Impfung einen vielfach höheren IgG2a- als IgG1-Antikörper-Titer induziert, was für eine T_{H}1-vermittelte Immunreaktion spricht. Dieses Ergebnis zeigte sich auch hier im zweiten Versuch bei der subkutanen Impfung. Bei intramuskulärer und intranasaler Immunisierung mit AdV-F_{syn} dagegen war die IgG2a / IgG1-Ratio kleiner. Während nach intramuskulärer Impfung noch mehr IgG2a- als 1gG1-Antikörper gebildet wurden, war das Verhältnis von IgG2a und IgG1 nach intranasaler Immunisierung recht ausgeglichen (Figur 7). Somit wird der T_{H}1-vermittelte Weg bei der Immunreaktion gegen RSV am stärksten bei der subkutanen Immunisierung angesprochen. Aber auch bei intranasaler Impfung ist das Verhältnis von T_{H}1 (IgG2a) und T_{H}2 (IgG1) ausgeglichen, was für eine balancierte Tₙ1 / T_{H}2-Antwort spricht. Somit wird der IgG-Antikörperklassen-Verteilung zufolge über keine der drei Administrationsrouten eine überschießende T_{H}2-Antwort erzeugt, was für eine Impfung gegen RSV bedeutsam ist.

Des Weiteren war bei allen drei mit AdV-F_{syn} geimpften Mäusegruppen von der Serumprobe nach erster Immunisierung zur Serumprobe nach zweiter Immunisierung bei beiden IgG-Antikörperklassen ein weiterer Titeranstieg vorhanden, wobei dieser bei intranasaler Immunisierung am geringsten und bei subkutaner Immunisierung am stärksten ausgeprägt war (Figur 7). Man kann daher feststellen, dass ein Boost bei AdV-F_{syn}-Impfung einen weiteren Antikörperanstieg induziert und somit für den Aufbau einer guten Immunität gegen RSV sinnvoll ist.

Auch im Neutralisationstest zeigte sich wieder, dass die Impfung mit AdV-F_{syn} eine sehr starke spezifische Serum-Antikörper-Antwort gegen RSV induziert: Während vor der ersten Immunisierung in allen Mäusegruppen keine neutralisierenden Antikörper gegen RSV nachgewiesen werden konnten und dieser Titer in der nicht-immunisierten Kontrollgruppe auch nicht anstieg, zeigte sich bei den drei mit AdV-F_{syn} geimpften Gruppen eine starke Erhöhung RSV-spezifischer neutralisierender Antikörper. Wie im IgG-ELISA und bei der RSV-qRT-PCR (Lungenhomogenat) waren auch hier Unterschiede abhängig von der Administrationsroute vorhanden. So führte die intranasale Immunisierung zur stärksten Erhöhung der neutralisierenden Aktivität, wobei der Titer um einen Faktor von circa 200 anstieg. Dabei war der Titeranstieg nach intranasaler Immunisierung 10fach stärker als nach intramuskulärer Impfung (hier fand sich ein Titeranstieg um einen Faktor von circa 20) und 6fach stärker als nach subkutaner Impfung (hier fand sich ein Titeranstieg um einen Faktor von circa 35). Somit wurde gezeigt, dass die Immunisierung mit AdV-F_{syn} unabhängig von der Administrationsroute (intranasal, intramuskulär, subkutan) eine sehr gute Antwort an systemischen neutralisierenden Antikörpern gegen RSV hervorruft, wobei die intranasale Route die stärkste Antwort induziert (Figur 8).

Weiterhin zeigte sich hier, wie auch schon im ELISA, dass die zweite Immunisierung einen weiteren Antikörperanstieg induziert. Die neutralisierende Aktivität war beim Serum nach zweiter Immunisierung im Vergleich zum Serum nach erster Immunisierung deutlich angestiegen, am stärksten bei der intranasal immunisierten Gruppe (etwa 14fach), am schwächsten bei der subkutan immunisierten Gruppe (etwa 2fach) (Figur 8).

Insgesamt fanden sich bei dem Ergebnis des Neutralisationstests stärkere Analogien zur qRT-PCR von den RNA-Isolaten aus dem Lungenhomogenat als zum ELISA. So korrespondierte der induzierte Titer an RSV-spezifischen neutralisierenden Antikörpern mit der Reduktion der RSV-Beladung in der Lunge, aber nicht mit dem Titer der im ELISA detektierten RSV-spezifischen Antikörper. Dies führt zu dem Schluss, dass der ELISA nicht nur die RSV-spezifischen neutralisierenden Antikörper detektiert, sondern vielmehr andere nicht-neutralisierende, gegen RSV gerichtete Antikörper. Da - wie aus dem Zusammenhang zwischen dem Titer an neutralisierenden Antikörpern und der Reduktion der RSV-Beladung in der Lunge ersichtlich ist - die RSV-spezifischen neutralisierenden Antikörper wesentlich an der Induktion des Schutzes vor RSV beteiligt sind, kann der Neutralisationstest besser als der ELISA zur Beurteilung des Schutzes nach Impfung herangezogen werden.

Bei der durchgeführten Immunisierungsstudie wurde mit der intranasalen Immunisierung das eindeutig beste Impfergebnis erzielt, da nach intranasaler Immunisierung mit AdV-F_{syn} einerseits der höchste Titer an neutralisierenden Antikörpern gefunden wurde und andererseits die RSV-Beladung in der Lunge am stärksten (nahezu vollständig) reduziert war. Somit ist bei Prime-Boost-Vakzinierung mit AdV-F_{syn} die intranasale Route zu favorisieren.

### Beispiel 12: Subkutane Immunisierung mit AdV-F_{syn} in verschiedenen Dosierungen (Dosiseskalation)

In diesem Experiment sollte der Einfluss der Impfstoffdosis auf die subkutane Immunisierung mit AdV-F_{syn} untersucht werden. Dazu wurden vier Gruppen von je 6 BALB/c-Mäusen jeweils subkutan mit Dosierungen von 2 x 10⁵ bis 2 x 10⁸ GTU (1 x 10⁷ bis 1 x 10¹⁰ OPU) AdV-F_{syn} immunisiert. Eine fünfte Gruppe mit ebenfalls 6 BALB/c-Mäusen wurde nicht immunisiert (Negativkontrolle). Das Zeitschema für Impfungen, Blutentnahmen und Challenge wurde von der ersten Immunisierungsstudie übernommen (siehe Beispiel 10).

Am Tötungstag (5 Tage nach Infektion) wurden BALs und Lungenhomogenate der Mäuse gewonnen. Nach Isolierung viraler RNA aus der BAL und Gesamt-RNA aus dem Lungenhomogenat wurden quantitative RT-PCRs durchgeführt, um die RSV-Beladung in den RNA-Isolaten zu bestimmen und eine Aussage über den Schutz der Tiere nach Impfung treffen zu können. Sowohl bei den RNA-Isolaten aus den BALs als auch bei den RNA-Isolaten aus den Lungenhomogenaten war die RSV-Beladung in der nicht-immunisierten Kontrollgruppe sehr hoch, wobei die Detektion der RSV-Kopien erneut in den RNA-Isolaten aus den Lungenhomogenaten deutlich sensitiver war.

Alle 4 immunisierten Mäusegruppen wiesen bei der qRT-PCR von den Isolaten aus den BALs einen guten Schutz auf, da bei keiner einzigen Maus eine RSV-Beladung nachweisbar war (Reduktion um einen Faktor von mindestens circa 95-fach) (Figur 9).

Bei der sensitiveren qRT-PCR von den Isolaten aus den Lungenhomogenaten dagegen war auch bei den immunisierten Mäusen eine RSV-Beladung nachweisbar, wobei die RSV-Kopienzahl bei allen vier Immunisierungsgruppen vergleichbar war. So fand sich in allen Immunisierungsgruppen eine durchschnittliche Reduktion der RSV-Beladung um einen Faktor von circa 340 von durchschnittlich rund 47000 Kopien auf durchschnittlich rund 140 Kopien (Figur 10). Somit waren alle immunisierten Mäuse unabhängig von der Impfstoffdosis gleichermaßen geschützt, wobei zwar ein guter, aber nicht vollständiger Schutz zu finden war. Das bedeutet, dass der durch eine subkutane AdV-F_{syn}-Impfung induzierte Schutz begrenzt ist und durch eine Erhöhung der Impfstoffdosis nicht weiter gesteigert werden kann. Einerseits ist demnach bei subkutaner Impfung zum Aufbau eines guten Schutzes eine niedrige Impfstoffdosis genauso wirksam wie eine hohe Impfstoffdosis, andererseits ist die subkutane Route bei der AdV-F_{syn}-Impfung nicht optimal, da hier (anders als bei der intranasalen Impfung im vorherigen Experiment) ein nur begrenzter Schutz induziert werden kann.

Die im Verlauf der Immunisierungsstudie gewonnenen Seren der Mäuse wurden im IgG-Antikörper-ELISA und im Neutralisationstest auf die Induktion systemischer RSV-spezifischer Antikörper getestet.

Vor der Immunisierung waren in keiner Mäusegruppe RSV-spezifische IgG1- oder IgG2a-Antikörper im ELISA nachweisbar. In der nicht-immunisierten Kontrollgruppe fand auch im Verlauf des Experiments kein Titeranstieg statt. Bei den vier subkutan mit AdV-F_{syn} geimpften Mäusegruppen dagegen fand sich ein deutlicher RSV-spezifischer Titeranstieg sowohl bei IgG1- als auch bei IgG2a-Antikörpern. Dabei war dieser Anstieg - anders als bei der RSV-qRT-PCR - von der Dosis des Impfstoffs abhängig. Bei den IgGl-Antikörpern fand sich nur ein geringer Unterschied in den Mäusegruppen, da - wie auch bereits in den bisherigen Immunisierungsstudien gezeigt wurde - bei der subkutanen Impfung grundsätzlich nur geringe IgG1-Antikörpertiter induziert werden, weil die Immunantwort T_{H}1-vermittelt verläuft. So fiel der Titeranstieg der RSV-spezifischen IgG1-Antikörper in allen vier Gruppen gleichermaßen schwach aus. Nur bei sehr hoher Impfstoffdosis konnte durch den Boost ein geringfügig stärkerer Anstieg induziert werden. Bei den IgG2a-Antikörpern dagegen wiesen die vier Mäusegruppen abhängig von der Impfstoffdosis einen deutlich variierenden Titeranstieg auf, sodass also hier eine direkte Abhängigkeit zwischen Impfstoffdosis und Titeranstieg besteht. So stieg sowohl nach der ersten Immunisierung als auch nach der zweiten Immunisierung der Titer abhängig von der Dosis unterschiedlich stark an. Während nach der ersten Immunisierung der Titer von einer Stufe der Dosierung zur nächsten (von Mäusegruppe zu Mäusegruppe) noch relativ linear, das heißt um jeweils ungefähr die gleiche Menge anstieg, zeigte sich nach dem Boost eine exponentielle Abhängigkeit, da die zweite Immunisierung bei geringer Dosis einen nur schwachen weiteren Titeranstieg bewirkte, bei hoher Dosis dagegen einen starken weiteren Titeranstieg. Somit führt die subkutane Immunisierung mit AdV-F_{syn} zu einem - anders als bei der RSV-Beladung entsprechend der qRT-PCR - Dosis-abhängigen Titeranstieg RSV-spezifischer Antikörper.

Während bei hohen Dosierungen die zweite Immunisierung zu einem starken weiteren Titeranstieg führte, wurde bei geringen Dosierungen durch den Boost kaum ein weiterer Titeranstieg bewirkt, sodass - was den RSV-spezifischen Antikörpertiter entsprechend des ELISAs betrifft - der Boost nur bei den beiden höchsten Dosierungen sinnvoll scheint (Figur 11).

Es wurde auch ein Neutralisationstest auf RSV-spezifische neutralisierende Antikörper durchgeführt. Vor Immunisierung und in der nicht-immunisierten Kontrollgruppe war jeweils kein Titer nachweisbar. Nach subkutaner Impfung mit AdV-F_{syn} dagegen wurden neutralisierende Antikörper gebildet. Dabei zeigte sich im Neutralisationstest ein anderes Bild als im ELISA. Hier sah man bereits nach der ersten Immunisierung eine klare Dosisabhängigkeit bei der Induktion neutralisierender Antikörper, wobei der Titer von Dosisstufe zu Dosisstufe exponentiell anstieg. So stieg der Titer bei der Dosis 2 x 10⁵ GTU (1 x 10⁷ OPU) um einen Faktor von circa 2,6, bei der Dosis 2 x 10⁶ GTU (1 x 10⁸ OPU) um einen Faktor von circa 3,3, bei der Dosis 2 x 10⁷ GTU (1 x 10⁹ OPU) um einen Faktor von circa 21, bei der Dosis 2 x 10⁸ GTU (1 x 10¹⁰ OPU) um einen Faktor von circa 36 an.

Während also im ELISA nach der ersten Immunisierung nur ein geringer Effekt der Dosiserhöhung zu beobachten war, wurden die neutralisierenden Antikörper durch eine hohe Impfstoffdosis wesentlich stärker induziert als durch eine niedrige Impfstoffdosis. Nach der zweiten Immunisierung dagegen war im Neutralisationstest (anders als im ELISA) keine Dosisabhängigkeit mehr zu sehen. Der Antikörpertiter stieg unabhängig von der Impfstoffdosis nach der zweiten Immunisierung in allen immunisierten Gruppen auf einen Maximalwert, wobei jeweils die höchste Serumverdünnung, bei der die Infektion durch RSV durch neutralisierende Antikörper zu 50% (IC50) gehemmt wird, bei durchschnittlich 1:295 lag. Das heißt, dass bei der zweifachen subkutanen Immunisierung mit AdV-F_{syn} nur ein maximaler Titer an neutralisierenden Antikörpern aufgebaut und dieser auch nicht durch eine weitere Dosiserhöhung gesteigert werden kann. Dieses Ergebnis deckt sich mit dem der qRT-PCR von RNA-Isolaten aus dem Lungenhomogenat, da auch hier bei allen immunisierten Mäusegruppen unabhängig von der Impfstoffdosis ein vergleichbar guter Schutz (ähnliche Reduktion der RSV-Beladung) gefunden wurde, was erneut zeigt, dass die im Neutralisationstest detektierten Antikörper wesentlich für den Schutz vor RSV-Infektion verantwortlich sind. Somit zeigt das Ergebnis des Neutralisationstests genauso wie die RSV-qRT-PCR, dass ein guter Schutz vor RSV-Infektion bei zweifacher subkutaner AdV-F_{syn}-Immunisierung auch durch eine niedrige Impfstoffdosis erreicht und durch Dosiserhöhung nicht weiter gesteigert werden kann. Wie aus dem Neutralisationstest ersichtlich ist, scheint dabei aber der Boost unbedingt nötig zu sein, um durch eine niedrige Impfstoffdosis einen genauso guten Schutz wie durch eine hohe Impfstoffdosis zu induzieren (Figur 12).

Bei der zweifachen subkutanen Immunisierung mit AdV-F_{syn} wird demnach nur ein begrenzter Schutz erzielt, da der maximal erreichte Wert bezüglich Reduktion von RSV-Beladung in der Lunge und induziertem Titer an neutralisierenden Antikörpern auch durch Dosiserhöhung nicht weiter gesteigert werden kann. Dieses Ergebnis verstärkt den aus der vorherigen Immunisierungsstudie gezogenen Schluss, dass die intranasale Route zu favorisieren ist. Des Weiteren scheint bei der subkutanen Immunisierung mit AdV-F_{syn}, da nach der zweiten Immunisierung in allen Mäusegruppen der gleiche maximale Titer an neutralisierenden Antikörpern erreicht und die RSV-Beladung in der Lunge gleichermaßen reduziert wurde, für den gleichen Impferfolg auch eine geringere AdV-F_{syn}-Dosis ausreichend zu sein, sofern eine Prime-Boost-Strategie gewählt wird.

### Beispiel 13: Expressionsteigerung von RSV-F Protein bei der Verwendung codonoptimierter Expressionssequenzen.

Nach Einbringen (Transfektion) der Expressionsplasmide pFwt (Expressionsplasmid mit der Wildtyp Sequenz des RSV-F Proteins), pIFwt (Expressionsplasmid mit der Wildtyp Sequenz des RSV-F Proteins mit einem zusätzlichen Intron vor dem offenen Leserahmen), pFsyn (Expressionsplasmid mit der codon-optimierten Sequenz des RSV-F Proteins) und pIFsyn (Expressionsplasmid mit der codon-optimierten Sequenz des RSV-F Proteins mit einem zusätzlichen Intron vor dem offenen Leserahmen) in Hep2 Zellen wurden diese 48 Stunden danach lysiert. Die Proteine des Zelllysats wurden mittels Gelelektrophorese der Größe nach aufgetrennt und anschließend auf eine Nitrocellulosemembran transferriert.

In den Zelllysaten wurde die Menge an exprimiertem RSV-F Protein bestimmt (Figur 14). Als Kontrollen dienten ein Plasmid ohne Fremdgen (Leerplasmid; pcDNA3.1), Zellen ohne Plasmid (Hep2) und mit RSV infizierte Zellen (Positivkontrolle; RSV). Die Ergebnisse zeigen, dass nur durch die Codon-Optimierung eine Expression erreichbar ist (Vergleich von pFwt oder pIFwt zu pFsyn oder pIFsyn).

Die Expression des codon-optimierten Konstrukts (pIFsyn) wurde durch serielle Verdünnung (1:10²bis 1:10⁴) des Zelllysates mit der Expression des Plasmids mit der ursprünglichen Sequenz (pIFwt) im unverdünnten Zelllysat (1:1) verglichen (Figur 15). Die Proteinmenge von RSV-F durch Expression des codon-optimierten Konstrukts liegt bei einer Verdünnung von 1 000-fach (1:10³) noch deutlich über der durch das Plasmid mit der ursprünglichen Sequenz. (kDa = Molekulargewicht in Kilodalton)

Die Ergebnisse zeigen, dass die erfindungsgemäße, codon-optimierte Nukleinsäuresequenz mit der SEQ ID NO:2 die Expression des RSV-F Proteins in Zellen ungewöhnlich stark steigert (Figur 14; Figur 15). Im Gegensatz zur bekannten Expressionssteigerungen von in der Regel 50 bis höchstens 100-fach, wird im Fall der Verwendung der erfindungsgemäßen Sequenz eine Steigerung der Expression von RSV F Protein von über 1000-fach gefunden. Im Gegensatz zu anderen viralen Proteinen, z.B. VSV-G Protein, lässt sich bei dem RSV-F Protein allein durch die Vorschaltung eines Introns keine Expressionsverstärkung erzielen.

## Patentansprüche

1. Nukleinsäuremolekül umfassend eine Nukleotidsequenz, die für ein Fragment des F-Proteins des Respiratorischen Syncytialvirus (RSV) kodiert, **dadurch gekennzeichnet, dass** die Nukleotidsequenz ein Fragment von SEQ ID NO:2 ist, wobei das Fragment
(i) mindestens 100 Nukleotide lang ist;
(ii) 3' und/oder 5' gegenüber der Nukleotidsequenz von SEQ ID NO: 2 verkürzt ist; und
(iii) ausreichende Immunogenität besitzt, um in einem Organismus eine Immunantwort auszulösen.

2. Nukleinsäuremolekül nach Anspruch 1, **dadurch gekennzeichnet, dass** das Fragment des F-Proteins des Respiratorischen Syncytialvirus (RSV) die RSV F-Protein Ektodomäne ist.

3. Vektor, **dadurch gekennzeichnet, dass** der Vektor das Nukleinsäuremolekül gemäß einem der Ansprüche 1-2 umfasst.

4. Vektor nach Anspruch 3, wobei der Vektor ein viraler Vektor oder ein Plasmid ist.

5. Vektor nach Anspruch 4, **dadurch gekennzeichnet, dass** der virale Vektor ein replikationsdefizienter, adenoviraler Vektor ist, der (a) ein Nukleinsäuremolekül gemäß einem der Ansprüche 1-2 und (b) eine Transkriptions- und/oder Translationskontrollsequenz in funktionaler Verknüpfung mit dem Nukleinsäuremolekül umfasst.

6. Immunogene Zusammensetzung, **dadurch gekennzeichnet, dass** die Zusammensetzung ein Nukleinsäuremolekül gemäß einem der Ansprüche 1-2 umfasst oder einen Vektor gemäß einem der Ansprüche 3-5 und einen pharmazeutisch annehmbaren Träger umfasst.

7. Nukleinsäuremolekül gemäß einem der Ansprüche 1-2 oder Vektor gemäß einem der Ansprüche 3-5 für die Verwendung als Impfstoff zur Impfung eines Subjekts gegen eine durch eine RSV-Infektion verursachte Erkrankung.

8. Verfahren zur Herstellung eines RSV F-Proteins, **dadurch gekennzeichnet, dass** das Verfahren (a) die Transfektion einer Wirtszelle mit einem Nukleinsäuremolekül gemäß einem der Ansprüche 1-2 und (b) die Expression des Nukleinsäuremoleküls in der Wirtszelle umfasst.

## Claims

1. Nucleic acid molecule comprising a nucleotide sequence which codes for a fragment of the respiratory syncytial virus (RSV) F-protein, **characterised in that** the nucleotide sequence is a fragment of SEQ ID NO: 2, the fragment
(i) being at least 100 nucleotides long,
(ii) being 3'- and/or 5'- shortened with respect to the nucleotide sequence of SEQ ID NO: 2, and
(iii) having sufficient immunogenicity to trigger an immune response in an organism.

2. Nucleic acid molecule according to claim 1, **characterised in that** the fragment of the respiratory syncytial virus (RSV) F-protein is the RSV F-protein ectodomain.

3. Vector, **characterised in that** the vector comprises the nucleic acid molecule according to either claim 1 or claim 2.

4. Vector according to claim 3, wherein the vector is a viral vector or a plasmid.

5. Vector according to claim 4, **characterised in that** the viral vector is a replication-defective adenoviral vector which comprises (a) a nucleic acid molecule according to either claim 1 or claim 2 and (b) a transcription and/or translation control sequence in functional linkage with the nucleic acid molecule.

6. Immunogenic composition, **characterised in that** the composition comprises a nucleic acid molecule according to either claim 1 or claim 2, or a vector according to any of claims 3-5, and a pharmaceutically acceptable carrier.

7. Nucleic acid molecule according to either claim 1 or claim 2, or vector according to any of claims 3-5, for use as a vaccine for vaccinating a subject against a disease caused by an RSV infection.

8. Method for producing an RSV F-protein, **characterised in that** the method comprises (a) the transfection of a host cell with a nucleic acid molecule according to either claim 1 or claim 2, and (b) the expression of the nucleic acid molecule in the host cell.

## Revendications

1. Molécule d'acide nucléique comprenant une séquence nucléotidique codant un fragment de la protéine F du virus syncytial respiratoire (VSR), **caractérisée en ce que** la séquence nucléotidique est un fragment de la SEQ ID N°2, dans laquelle ledit fragment
(i) a une longueur d'au moins 100 nucléotides ;
(ii) est réduit en 3' et/ou 5' par rapport à la séquence nucléotidique de la SEQ ID N°2 ; et
(iii) possède une immunogénicité suffisante pour déclencher une réponse immunitaire dans un organisme.

2. Molécule d'acide nucléique selon la revendication 1, **caractérisée en ce que** le fragment de la protéine F du virus syncytial respiratoire (VSR) est l'ectodomaine de la protéine F du VSR.

3. Vecteur, **caractérisé en ce qu'**il comprend la molécule d'acide nucléique selon l'une des revendications 1 à 2.

4. Vecteur selon la revendication 3, **caractérisé en ce que** le vecteur est un vecteur viral ou un plasmide.

5. Vecteur selon la revendication 4, **caractérisé en ce que** le vecteur viral est un vecteur adénoviral déficient en réplication, qui comprend (a) une molécule d'acide nucléique selon l'une des revendications 1 à 2 et (b) une séquence de contrôle de la transcription et/ou de la translation en relation fonctionnelle avec la molécule d'acide nucléique.

6. Composition immunogène, **caractérisée en ce que** la composition comprend une molécule d'acide nucléique selon l'une des revendications 1 à 2 ou un vecteur selon l'une des revendications 3 à 5 et un support acceptable pharmaceutiquement.

7. Molécule d'acide nucléique selon l'une des revendications 1 à 2 et un vecteur selon l'une des revendications 3 à 5 pour l'utilisation en tant que vaccin pour la vaccination d'un sujet contre une maladie provoquée par une infection au VSR.

8. Procédé de fabrication d'une protéine F du VSR, **caractérisé en ce que** le procédé comprend (a) la transfection d'une cellule hôte avec une molécule d'acide nucléique selon l'une des revendications 1 à 2 et (b) l'expression de la molécule d'acide nucléique dans la cellule hôte.
